(19) Europäisches Patentamt
European Patent Office
Office européen des brevets

(11) EP 0 889 691 B1

(12) EUROPEAN PATENT SPECIFICATION

(45) Date of publication and mention of the grant of the patent:
**29.01.2003 Bulletin 2003/05**

(21) Application number: **97927593.0**

(22) Date of filing: **25.03.1997**

(51) Int Cl.[7]: **A01N 35/02**, A01N 65/00, C12N 15/82, A01N 3/02, A01N 31/04, A01N 37/10, A01N 37/44 // (A01N35/02, 65:00, 59:04, 35:02)

(86) International application number:
**PCT/US97/05400**

(87) International publication number:
**WO 97/035471 (02.10.1997 Gazette 1997/42)**

(54) **USE OF AROMATIC ALDEHYDES AS PESTICIDES**

VERWENDUNG VON AROMATISCHEN ALDEHYDEN ALS PESTIZIDE

UTILISATION D'ALDEHYDES AROMATIQUES COMME PESTICIDES

(84) Designated Contracting States:
**AT BE CH DE DK ES FI FR GB GR IE IT LI LU MC NL PT SE**
Designated Extension States:
**AL LT LV RO SI**

(30) Priority: **25.03.1996 US 624700**

(43) Date of publication of application:
**13.01.1999 Bulletin 1999/02**

(73) Proprietor: **PROGUARD, INC.**
**Suisun City, CA 94585 (US)**

(72) Inventors:
- **EMERSON, Ralph, W.**
  **Davis, CA 95616 (US)**
- **CRANDALL, Bradford, G., Jr.**
  **Davis, CA 95616 (US)**

(74) Representative:
**Smulders, Theodorus A.H.J., Ir. et al**
**Vereenigde**
**Postbus 87930**
**2508 DH Den Haag (NL)**

(56) References cited:

| | |
|---|---|
| **EP-A- 0 692 191** | **WO-A-94/08036** |
| **WO-A-95/27790** | **WO-A-96/20596** |
| **WO-A-96/41528** | **DE-C- 4 423 022** |
| **FR-A- 2 405 650** | **FR-A- 2 529 755** |
| **US-A- 4 978 686** | |

- **DATABASE WPI Section Ch, Week 8235 Derwent Publications Ltd., London, GB; Class C03, AN 82-73641E XP002040222 & JP 57 120 501 A (SAOTOME K) , 27 July 1982**
- **CHEMICAL ABSTRACTS, vol. 96, no. 13, 29 March 1982 Columbus, Ohio, US; abstract no. 99381, K.SITARAMAIAH ET AL.: "Effect of growth regulators, phenolics and aromatic acid on root-knot severity (Meloidogyne incognita and M.javanica) on tomato" XP002040221 & Z.PFLANZENKR.PFLANZENSCHUTZ, vol. 88, no. 11, 1981, pages 651-654,**
- **BIO/TECHNOLOGY, vol. 10, November 1992, pages 1436-1445, XP002032892 LAMB C J ET AL: "EMERGING STRATEGIES FOR ENHANCING CROP RESISTANCE TO MICROBIAL PATHOGENS"**
- **DATABASE WPI Section Ch, Week 9507 Derwent Publications Ltd., London, GB; Class C05, AN 95-048734 XP002040223 & JP 06 329 514 A (TAKEDA CHEM IND LTD) , 29 November 1994**
- **DATABASE WPI Section Ch, Week 9324 Derwent Publications Ltd., London, GB; Class C03, AN 93-191413 XP002040224 & JP 05 117 125 A (DAINICHISEIKA COLOR & CHEM MFG) , 14 May 1993**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 889 691 B1

## Description

## INTRODUCTION

### Field of the Invention

The present invention is related to the biocontrol of plant pathogens through nutritional mediation. The invention is exemplified by the use of nonphytotoxic compositions containing balsam compounds to control growth of fungi and parasitic insects, including sap-sucking insects, which colonize the surfaces of plant parts and tissues.

### Background

The surfaces of plant parts such as roots and leaves are colonized by a variety of organisms, many of which are dependent upon the host plant as a source of nutrients. The colonizing organisms include pathogenic fungi and sap-sucking insects; both groups are capable of inflicting severe damage to the host plant, including stunting the growth of the host plant and decreasing plant productivity, to killing the host plant.

Fungi pathogenic for plants are many and diverse. They occur in most groups of fungi. A few, such as rusts, *Uredinales*, and powdery mildew and downy mildew, *Erysiphacea and Peronosporacea,* are obligate parasites. Generally, a particular rust or mildew is associated with specific host plants which elaborate nutrients required by the pathogen. As an example, rust, caused by *Phragmidrium mucronatrum,* is an important fungal disease associated with roses; it produces bright orange pustules on the underside of rose leaves and pale yellow spots on the top. Powdery mildew, caused by *Sphaerotheca pannosa* (Wallr. ex. Fr.) Lev var. *rosae* Woronichine also is associated with roses and is probably the most widely distributed and serious disease of glasshouse, garden, and field-grown roses alike.

Pathogenic insects which infest plants include those insect species which are symbiotic with bacteria, such as aphids, leaf hoppers, and white fly; the host insect cannot survive without the symbionts. As an example, aphids (*homoptera*) possess symbiotic bacteria of the genus *Buchnera* in cells called mycetocytes within the hemocoel. The bacteria are transmitted directly from the maternal aphid to her offspring and aposymbiotic aphids do not occur naturally. The bacteria may provide lipids which are required for embryogenesis of the host insect but which are absent or in low concentrations in phloem sap in plants infected by the insects.

The plant pathogens include the grape phylloxera (*Daktulosphaira vitifoliae*), an aphid-like insect, and nematodes. Phylloxera is native to the United States east of the Rocky Mountains, where it lives on native wild species of grapes, which have evolved resistance to the feeding of the insect. The European grape *(Vitis vinifera)*, which is used to produce wine, evolved in western Asia and has no resistance to phylloxera. Stem and bulb nematode infestations *(Ditylenchus dipsaci*) have been recorded in all the major agricultural regions in California. This wide distribution probably reflects its spread on such infested planting material as garlic cloves. Wherever such infested material is grown, the nematode may be introduced. *Ditylenchus dipsaci* can be found parasitizing a wide range of cultivated and wild plants. Nematodes produce galls in infected tissue. In addition to the disturbance caused to plants by the nematode galls themselves, damage to infected plants is increased by certain parasitic fungi, which can easily attach to the weakened root tissues and the hypertrophied, undifferentiated cells of the galls. Moreover, some fungi, for example, *Pythium, Fusarium*, and *Rhizoctonia*, grow and reproduce much faster in the galls than in other areas of the root, thus inducing an earlier breakdown of the root tissues.

A variety of pesticide compositions are used for controlling plant pathogens. For example, protective fungicidal sprays on a 6-7 day schedule for both rust and powdery mildew when environmental conditions favor disease development are the typical means of control. Two frequently used systemic fungicides are benomyl and triforine. However, the cost of fungicides for control of powdery mildew is high: in cut rose crops the cost of treatment in the State of California is several million dollars a year.

The older fungicides include inorganic compounds such as copper and sulfur and the organic protectants such as thiram, captam, maneb, and chlorotholonil. These compounds act only at the surface of the plant and must be present at or before appearance of the fungal pathogen in order to prevent infection. These older fungicides are multisite inhibitors; i.e., they affect many metabolic activities in a fungus. The newer fungicides tend to be highly effective organic systemics such as benzimidazoles, sterol biosynthesis inhibitors, carboxanilides, and phenylamides which act internally as well as at the plant surface. In contrast to the older surface protectants, the systemic fungicides are generally effective at much lower dosages and can cure established fungal infections, a critical factor in disease management. The systemic fungicides usually act at a single target site in the fungus, interfering with specific metabolic processes that are necessary for production of all new cell material required for growth, maintenance, and virulence of the fungal organism. These preparations typically are effective only against fungal pathogens.

Current methods of chemical control for certain above-ground pests (*e.g.*, spider mite, aphids, silverleaf white fly, leaf hoppers) include those which combine two insecticides from different chemical classes, for example, combining

a synthetic pyrethroid with an organophosphate or organochlorine insecticide. Soil fumigants have been a popular treatment for soil pests (nematodes, phylloxera). Use of certain highly effective types of insecticides and fumigants has sharply decreased in recent years due to cancellations of public regulatory agency registrations, or refusals of re-registrations, of products. However, due to a dearth of effective pest-control agents, some products which are known to be unsafe, such as methyl bromide, are being approved.

The wide-spread use of pesticides has resulted in the development and evolution of resistant pathogens, as well as growing environmental and health care concerns. A highly visible ecological-environmental activist community and public regulatory agencies have resulted in fewer and fewer pesticide registrations and, consequently, less related research and development. It therefore is of interest to identify and/or develop, "biorational" fungicides which have lower animal and environmental toxicities and which do not exhibit significant phytotoxicity at the concentrations used to control pathogenic fungi and insects.

Relevant literature

A method of protecting crops from attack of insect pests, microorganisms and pathogenic microbes using a composition comprising cinnamic aldehyde and requiring an antioxidant is disclosed in U.S. Patent No. 4,978,686. Protection of crops against pathogenic microorganisms and insect pests by applying an aqueous composition containing a cinnamaldehyde is disclosed in French patent application 2529755. U.S. Patent No. 2,465,854 describes an insecticidal composition containing a cinnamic aldehyde derivative. Control of *Verticillium* using cinnamaldehyde in the substrate in which mushrooms are grown is disclosed in U.S. Patent No. 5,149,715.

PCT/US95/17053 discloses a method for controlling growth of pathological organisms on a plant by providing the plant surface with an aqueous nonphytotoxic formation comprising 1-50 g/l of one or more aromatic aldehyde such as cinnamic aldehyde and coniferyl aldehyde.

Reweri *et al.* describe induction of systemic resistance to powdery mildew in cucumber by phosphates. *Biol. Agric. and Hort.* (1993) 9:305-315. Horst and Kawamato disclose the effect of sodium bicarbonate and oils on the control of powdery mildew and black spot on roses. *Plant Disease,* March 1992, p.247. Sodium bicarbonate and severely solvent refined light paraffinic petroleum oil have been used to control black spot and powdery mildew. Ziv *et al.*, *Hort. Science* (1993) 28:124-126.

Elad *et al*. disclose the effect of film-forming polymers on powdery mildew of cucumber. *Phytoparasitica* (1989), 17:279-288. Hagiladi and Ziv disclose the use of an antitranspirant for the control of powdery mildew in the field. *J. Environ. Hortic.* (1986), 4:69-71. Macro *et al*. disclose control of powdery mildew of roses with antitranspirant coating polymers. *Phytoparasitica* (1994) 22:19-29. Paulus and Nelson disclose use of flusilarzol, myciobutanil, fenarimol, pentonazote, and diniconazole for controlling powdery mildew and rust in roses. *Calif. Agric.* 1988, 42:15.

U.S. Patent No. 4,402,950 describes the deactivation of viruses inside living human and animal organisms by application of a terpene obtainable from aromatic plants by steam application. The terpenes cited are: black pepper oil, cinnamon flour oil, cardamon oil, linallyl acetate, cinnamic aldehyde, safrol, carvon and cis/trans citrao. U.S. Patent No. 4,477,361 describes a cinnamic compound containing an anti-microbial surfactant which is rendered substantive to the surface being washed.

References relating to anti-microbial properties of various saponins either alone or in combination with other agents include the following: JP2157205, DE3724595, JP61065802, JP61007290. Inhibiting cinnamyl alcohol dehydrogemase (CAD) activity in transgenic plants has been proposed as a method of reducing lignin synthesis in plants and thereby improving the digestibility of fodder crops (WO 93/05159).

## SUMMARY OF THE INVENTION

The present invention provides compositions and methods for controlling pathogenic organisms on plants, as well as providing seeds, seedlings and plants substantially free of plant pathogens through nutritional mediation. The method includes the step of administering to a plant an antipathogenic composition which contains an agent which increases accumulation of aromatic aldehydes in the plant or increases cinnamic acid in the plant. The antipathogenic composition can be administered directly to the plant or the plant can be transformed with a vector containing a nucleotide sequence encoding the agent under the control of a promoter functional in the plant. The antipathogenic composition comprises one or more of an aromatic aldehyde, an ester or an acid or another aromatic compound. The invention also provides a nonphytotoxic formulation comprising balsam. The invention is used to provide sustained resistance to a variety of pathogenic organisms which colonize plant tissues and/or parts.

## DESCRIPTION OF THE FIGURES

Figure 1 shows the results of a bioassay of different concentrations of Storax against the melon aphid on chrysanthemum leaves.

Figure 2 shows the results of a bioassay of 0.6% Storax alone, 0.6% Storax plus 0.1% cinnamic aldehyde (CNMA) or α-hexyl cinnamic aldehyde (HCA) against the melon aphid on chrysanthemum leaves.

Figure 3 shows the results of treatment with 5% α-hexyl cinnamic aldehyde in different formulation on aphid mortality.

## DESCRIPTION OF SPECIFIC EMBODIMENTS

Seeds, seedlings, plants, and plant parts such as fruit substantially free of pathogenic organisms such as fungi and sapsucking insects are provided together with a method to biocontrol pathogen infestations on plants using aromatic aldehydes. By "biocontrol" is intended control of plant pathogens via direct antipathogenic activity and/or via indirect activity such as induced resistance of the host plant to pathogen infestation which increases accumulation of an aromatic aldehyde or increases cinnamic acid in the plant. A fungus and/or insect colonizing a surface of a plant part such as a leaf, root, or flower part, or a tissue such as xylem or phloem, is contacted with a natural product. By "colonizing" is intended association of a microorganism or insect with a plant part or tissue from which the pathogen derives nutrients, typically essential nutrients such as amino acids, particularly methionine. By "natural product" is intended an organic compound of natural origin that is unique to one organism, or common to a small number of closely related organisms, and includes secondary metabolites of fungi and chemicals produced by plants. The natural products can be isolated from a natural source, be wholly or partially synthetic, or be produced by recombinant techniques either by the host plant or by a transformed microorganism.

The method of the subject invention provides a susceptible plant with increased resistance to pathological microorganisms. The invention is carried out by administering to a plant a nonphytotoxic composition comprising an antipathogenic agent, to impair the growth and viability of a pathological microorganism which colonizes a plant surface or a plant part. The antipathogenic agent for example is a compound in the biosynthetic pathway for cinnamic aldehyde, or a compound that down regulates the expression of enzymes which metabolize precursor compounds in the biosynthetic pathway to cinnamic acid. The composition typically comprises at least one of cinnamic aldehyde, α-hexyl cinnamic aldehyde, cinnamic acid, a cinnamic ester of α- and/or β-steresin, and a cinnamate. The composition also can be added to a substrate in which a plant is growing or is to be growing. The amount of antipathogenic agent that is applied either to the plant itself or to the plant pathogens will depend upon the degree of infestation and to some extent upon the formulation and the specific compounding used and therefore must be empirically determined for best results. By "antipathogenic" is intended a pesticide, *i.e.* a formulation which is effective for controlling the growth of pathogens and can involve killing the pathogen and/or slowing or arresting its proliferation. Pathogens include insects, fungi and other microorganisms which negatively affect the plants which they colonize.

The compositions and methods of the subject invention offer several advantages over existing compositions and methods. An aromatic aldehyde, cinnamic aldehyde, has been reported to exhibit antifungal properties, but it has not previously been used on plants in an aqueous emulsion or solution in the absence of an anti-oxidant. As an example, U.S. Patent Application No. 4,978,686, discloses that an anti-oxidant is required for use with cinnamic aldehyde for a composition which is used for application to crops. Anti-oxidants are expensive, accordingly significant cost benefits are realized with the subject invention. In addition, a single application of one or more aromatic compound either directly or indirectly is sufficient for long term protection of the plant host from pathogenic organisms, including both rust and powdery mildew, and is effective at lower concentrations than has been reported previously.

The formulations of the subject invention also are effective against pests known to be resistant to conventional treatments, including such pests as thrips, melon aphid and citrus aphid. Phytotoxicity of the formulation also is decreased due to the lower concentrations of aromatic compound which are used and the lesser number of applications required. The subject formulations also provide for effective control of both fungi and insects, eliminating the need for application of multiple agents. In particular situations, such as where an insect damages a plant part or tissue and a secondary fungal disease develops, this aspect of the invention is particularly advantageous. The long term control of pathogenic organisms results in a healthier plant and an improved yield of produce by the host plant as compared to untreated plants; the lower concentrations and lesser numbered doses, generally single dose, of antipathogenic agents not only decrease the likelihood of damage to the plant or its crop but also decrease the likelihood of any adverse side effects to workers applying the pesticide, or to animals, fish or fowl which ingest the tissues or parts of treated plants. Another advantage is that the aromatic aldehydes in particular have positive organoleptic and olfactory properties which in some cases may improve the flavor and/or smell of treated products. Cinnamic aldehyde has a cinnamon

odor. The odor of α-hexyl cinnamic aldehyde (HCA) is described as floral or jasmine-like with some herbaceous character (Technical Data Sheet).

A number of the aromatic and aliphatic aldehydes which find use in the subject invention, such as α-hexyl cinnamaldehyde, benzaldehyde, acetaldehyde, cinnamaldehyde, piperonal, and vanillin are generally regarded as safe (GRAS) synthetic flavoring agents (21 CFR § 172.515), as is Storax (21 CFR § 172.510) which has been approved for food use. HCA was in public use before the 1950's and today is widely used in consumer preparations (soaps, detergents, creams, lotions, perfumes) (Monographs on fragrances raw materials. *Food Cosmet. Toxicol*. 12: suppl., 915, 1974). HCA was granted GRAS (generally recognized as safe) status by FEMA (Flavoring Extract Manufacturers' Association. Survey of flavoring ingredient usage levels. No. 2569. Fd. Technol., Champaign, 19: (part 2) 155, 1965) in 1965 and is approved by the US FDA for use in food (21 CFR § 121.1164). The Council of Europe included HCA in the list of admissible artificial flavoring substances at a level of 1 ppm (Council of Europe. Natural and Artificial Flavouring Substances. Partial Agreement in the Social and Public Health Field. Strasbourg, List A(1), Series 1, no. 129, p. 55, 1970). Various of these compounds have been reported to have inhibitory activity against *C. botulinum* spore germination. Bowles and Miller, G. *Food Protection* (1993) 56: 788-794. The compounds of interest are used with other compounds, including balsam, such as balms and storax, particularly for increasing pesticide effectiveness of other aromatic compounds and thereby reducing the phytotoxicity of the formulation. Many balsams are used in medicine, for example, Canada B., B. of Copaiba, Gurjum B., Mecea B., B. of Peru, and Tolu B.). Surfactants such as the Tweens (polysorbates) which can be used as emulsifiers are already used as food additives, as is saponin (which also has GRAS status).

Another advantage of the subject formulations is that formulation residuality can be managed. This is of great benefit when short term residuals are desired for integrated pest management programs with beneficial insects. In addition, the formulations are effective against pests such as those which are resistant to other agents. Reentry time to the greenhouse also is not an issue. Typically the formulations are rapidly lethal to a target organism; this is a particularly valuable characteristic when coupled with no reentry time, (for example, no loss of cut flower inventories).

An additional advantage of the subject formulations and methods is that treatment not only provides long-lasting protection against pests, but also is effective at a site on the plant remote from the point at which the subject formulations are applied. For example, foliar application of the subject formulations is effective against pathogens that colonize relatively remote and inaccessible regions of the plant, such as the roots and meristems. This remote effect occurs because the aromatic compounds and/or metabolic products such as cinnamic acid are transported in the plant vascular system, which allows for long distance transport of the compounds within living plants, and/or because application of the subject formulations induces systemic acquired resistance (SAR). SAR occurs in plants in response to infection, particularly by necrotizing pathogens, and provides enhanced resistance to subsequent attacks by the same or even unrelated pathogens. SAR provides long-term (weeks to months) protection throughout the plant (systemic) against a broad range of unrelated pathogens. Examples of defense response induced in plant cells include the synthesis of plant cell structural components such as cutin suberin, callose and lignin, chemical defense compounds such as $H_2O_2$, and anti-bacterial or anti-fungal compounds such as tannins and phytoalexins.

The method of introducing the active ingredient(s) of the formulation into the target organism can be by direct ingestion by the pest organism from a treated plant surface, or by feeding of a pest organism on a nutrient-providing surface of a host entity, which is colonized by the target pest organism. The host tissue or part either contains or has on its surface the antipathogenic agent. The presence of the anti-pathogenic agent on a nutrient-providing surface of a host plant can be a result of direct contact of the antipathogenic agent with the plant part, such as by foliar application, or it can be by elaboration from the host plant as a result of induction of systemic resistance as a secondary effect to prior treatment of the plant with the antipathogenic agent, or as a result of genetic modification of the host plant.

The antipathogenic agents include those having a formula shown in (1) below:

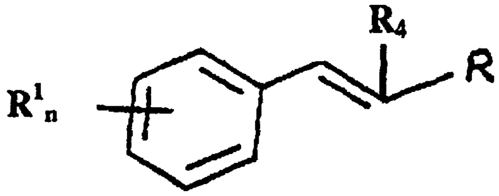

(1)

wherein R represents -CHO, $CH_2OH$, -COOH, or $-COOR_5$; n is an integer from 0 to 3; each $R^1$ represents -OH or an organic substituent containing from 1 to 10 carbon atoms and from 0 to 5 heteroatoms, wherein the total number of carbon and heteroatoms in all $R^1$ substituents of said compound is no more than 15; $R_4$ represents -H or an organic constituent containing from 1 to 10 carbon atoms; and $R_5$ represent an organic substituent containing from 1 to 10

carbon atoms and from 0 to 5 heteroatoms.

A preferred formulation is shown in formula (2) below:

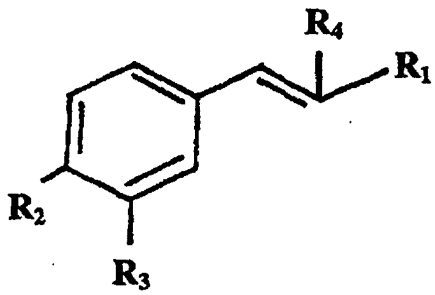

(2)

wherein $R_1$ represents-CHO, $R_2$ represents -H, -OH or an organic substituent containing from 1 to 10 carbon atoms, and $R_3$ represents -H, a methoxy group or organic substituent containing from 1 to 10 carbon atoms, and $R_4$ represents -H, or an organic substituent containing from 1 to 10 carbon atoms. Of particular interest are aromatic aldehydes. Examples of aromatic aldehydes of use in the present invention are cinnamic aldehyde ((3) below):

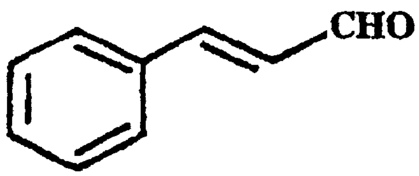

(3)

and coniferyl aldehyde ((4) below):

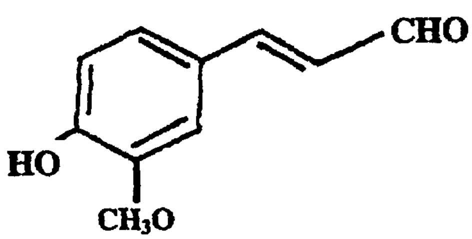

(4)

Other compounds of interest include analogs of the compound of formula (1) such as cinnamic esters, aldehydes and acids, as well as compounds substituted at the α position with an alkyl, such as a hexyl group, or a branched alkyl group such as an amyl group. Generally the group at the alpha position is from C-5 to C-10. Such compounds include α-hexyl cinnamaldehyde and α-amyl cinnamaldehyde. The chemical structure of α-hexylcinnamic aldehyde (HCA) is shown in (5) (below):

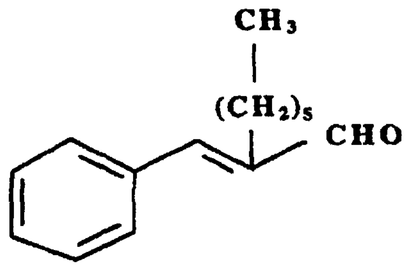

(5)

The Chemical Abstracts Service (CAS) name of HCA is 2-(phenylmethylene) octanal and the CAS Registry Number is [101-86-0]. The compound is also described by the chemical name of 2-hexyl-3-phenyl-2-propenal. The compound's formula is $C_{15}H_{20}O$ and the molecular weight is 216.3. HCA is a low to moderately volatile compound, having a vapor pressure of 70 x $10^{-5}$ mm Hg at 25°C. Its parent compound, cinnamic aldehyde, has a vapor pressure approximately 40 times higher (2970 x $10^{-5}$ mm Hg at 25°C). For comparison purposes, the insect repellant N,N-diethyl-m-toluamine has a slightly higher vapor pressure (167 x $10^{-5}$ mm Hg at 25°C) (Reifenrath, W.G. (1995) *Volatile Substances. Cosmetics and Toiletries,* 110: 85-93).

The aromatic and aliphatic aromatics of the subject invention can be prepared by various synthetic methods known to those skilled in the art. For example, *see,* J. March, ed., Appendix B, *Advanced Organic Chemistry: Reactions, Mechanisms, and Structure*, 2nd Ed., McGraw-Hill, New York, 1977. Cinnamaldehyde can be prepared synthetically, for example, by oxidation of cinnamyl alcohol (Traynelis *et al*., *J. Am. Chem. Soc*. (1964) 86:298) or by condensation of styrene with formylmethylaniline (Brit. patent 504,125). The subject aldehydes also can be obtained by isolation from natural sources. For example, cinnamaldehyde can be isolated from woodrotting fungus, *Stereum subpileatum*. Birkinshaw *et al*., *Biochem. J*. (1957) 66:188.

HCA can be synthesized as described, for example, in USPN 5,055,621. On a laboratory scale, HCA can be synthesized by reaction of benzaldehyde with octanal under a nitrogen atmosphere (aldol condensation) (Personal Communication, Eric Walborsky, Firmenich Chemical Manufacturing Center, Port Newark, New Jersey). The reaction is conducted in a stirred flask charged with methanol, 309 ppm diphenylamine, potassium hydroxide and benzaldehyde. Following the slow addition of octanal, the reaction mixture is brought to a pH of 7.5-9.5 with acetic acid. Following evaporation of methanol and a wash of the reaction mixture with water, the organic phase is transferred to a distillation unit. Approximately 20-24% of the pot charge is removed as benzaldehyde and "lights", with the remaining distillate constituting α-hexylcinnamic aldehyde "heart cut." The "heart cut" is subjected to an additional fractionation, in which 1-5% (by weight) of the material is removed in "light" fractions, depending upon odor evaluation. The final product is a light yellow oil having a specific gravity of 0.955-0.965 at 20°C, a refractive index of 1.548-1.562 at 20°C, a boiling point of 305°C at 1 atmosphere, and a melting point of 26°C.

HCA also can be obtained from Firmenich; their product is composed principally of the (E)-cis isomer (93.8% maximum), and the (Z)-trans isomer (6% maximum). Among minor components is the self aldol condensation product of octanal (1-1.5% (Personal Communication, June Burkhardt, Firmenich, Plainsboro, New Jersey). The commercial product is stabilized with the addition of 0.04% 2, 6-di-tert-butyl-p-cresol (butylated hydroxytoluene or BHT), which serves as an anti-oxidant (Technical Data Sheet, Hexylcinnamic aldehyde 907600, Revision 853, Firmenich Inc., Plainsboro, New Jersey). HCA can be isolated from rice where it has been reported to occur naturally. (Givaudan-Roure Index, Givaudan-Roure Corporation, Clifton, New Jersey, 1994, p. 89).

The subject aromatic compounds can be used either alone or in combination with other active or inactive substances. In some instances, the efficacy of the formulation can be increased by adding one or more other components, *i.e*., a compound other than a compound of formula (1), to the formulation. It is preferable that the additional component(s) minimize phytotoxicity of a particular formulation while increasing the antipathogenic effect of the formulation.

Especially preferred is the use of a "synergist," which is component that, by virtue of its presence, increases the desired effect by more than an additive amount. The concentration of one or more of the other formulation ingredients can be modified while preserving or enhancing the desired phytotoxic and antipathogenic effect of the formulation. Of particular interest is the addition of components to a formulation to allow for a reduction in the concentration of one or more other ingredients in a given formulation while substantially maintaining efficacy of the formulation. Combination of such a component with other ingredients of the formulation can be accomplished in one or more steps at any suitable stage of mixing and/or application. A Balsam is a resinous mixture of varying composition obtained from several species of evergreen trees or shrubs; it generally contains oleoresins, terpenes, and usually cinnamic and benzoic acids.

An example of a synergist which finds use in the subject invention is a balsam. Any of the balsams can be used, which include cinnamon compounds such as cinnamic ester, phenopropyl cinnamate and free cinnamic acid. Of particular interest is Storax (CAS Number 8046-19-3; also known as Styrax) obtained from the trunk of *Liquidamber orientalis* Miller and American Storax from *Liquidamber sytraciflua.* The storax obtained from *L. orientalis* Miller is known as Levant Storax. Another storax of interest is American Storax from *L. sytraciflua.* Its components are provided in Merck (8778) and in Laung & Foster Encyclopedia of Common Natural Ingredients (Second Edition), Wiley Interscience. Storax is in the U.S. P. and regular status has been approved for food use (21 CFR § 172.510). The components of Storax are *Constit.* 35-50% α- and β-storesin and its cinnamic ester; 5-10% styrene; 10% phenylpropyl cinnamate; small amounts of ethyl cinnamate; benzyl cinnamate; 5-15% free cinnamic acid; atyrene; 0.4% levoratatory oil; $C_{10}H_{16}0$, and traces of vanillin. The balsam can be combined with one or more cinnamic aldehyde, such as cinnamic aldehyde or α-hexyl cinnamic aldehyde. To obtain the storax, the bark of the tree is bruised or punctured in the early summer, stimulating formation of balsam-secreting ducts. In autumn the balsam saturated bark is pealed off and pressed. The residual bark is boiled in water and pressed again to obtain a second quantity of balsam. When *Liquidamber sytraciflua* is the source of balsam, the exudate (balsam) collects in natural pockets between the wood and the bark and can be located by excrescenes on the trunk. Probable balsams also can be used which produce a formulation having a desired antipathogenic and/or phytotoxic effect and are considered equivalence of the invention. Generally, an effective amount of storax when used in combination with an aldehyde such as cinnamic aldehyde (0.1%) or α-hexyl cinnamic aldehyde (at 0.1%) is 0.1% to 2%, preferably less than 1%, or preferably 0.6% or less. The amount to use of particular components for various applications can readily be determined using an appropriate bioassay for the target organism using methods known to those people skilled in the art target organism.

Of particular interest is the addition of adjuvants to a formulation. By "adjuvant" is intended a substance added to a formulation to aid the operation of the main ingredient. A spray adjuvant performs this function in the application of an agricultural chemical. An effective spray adjuvant may be formulated to contain one or more surfactants, solvents or co-solvents. Systems containing surfactants. water and oily components have many other possibilities of forming ordered phases; the surfactant can organize itself into aggregates of various shapes to create micelles, with a first order phase as one of the possibilities. The surfactant also can collect at the interface between interpenetrating oil and water phases to create a microemulsion. For example, the formulation can be rendered substantive by including an emulsifier such as Tween 80. Other detergents which can be used include anionic detergents such as those described in U.S. Patent Application No. 4,978.686. Generally, detergents and other agents used in the formulation do not detract from the pesticide properties of the aromatic aldehydes but do increase the substantive properties of the formulation *(see* for example, U.S. Patent Application No. 4,477,361) and may improve the pesticide properties.

A preferred surfactant for pesticides is one or more saponin, which can be derived from any of a variety of sources. Saponins can not only be used as an adjuvant but also as a surfactant and for reducing phytotoxicity and/or increasing the efficacy of the aromatic compound used. Saponins are a class of compounds, each consisting of a sapogenin portion and a sugar moiety. The sapogenin can be a steroid or a triterpene and the sugar moiety can be glucose, galactose, a pentose, or a methylpentose. S. Budavari, ed., *The Merck Index,* 11th ed., Merck & Co., Inc., Rahway, N.J., 1990, p. 1328. The saponins for use in the present invention can be produced and/or isolated from various plant parts including fruit, leaf, seed and/or root, using means known in the art, from a variety of sources including the various plants known to produce them, ranging from yucca, quillaja, agave, tobacco, licorice, soybean, ginseng and asparagus to aloe woods. Saponins for use with the present invention are preferably non-toxic to humans and higher animals at the concentrations used. Most preferably the saponin for use in the present invention is non-toxic food grade, the preferred source being yucca plants. Preferably saponins are from *Yucca schidigera* or *Y. valida* and their equivalents. For both phytotoxicity control as well as toxicological safety, preferred saponins are from *Yucca spp.* The saponins generally are prepared by a cold press extraction process and the resulting liquid extract analyzed by HPLC for saponin concentration. The yucca fiber also can be used; it is typically sundried, mulled and sized by screening.

A variety of structurally related saponins are known, the most variable structural feature being the glycosylation pattern. Saponins also may contain additional modifications, such as the sarasaponins which are saponins with a steroid attached, and saponin structure can be modified by any number of enzymatic, chemical and/or mechanical means known in the art. Nobel, Park S., *Agaves,* Oxford Univ. Press. New York, 1994, pp. 42-44. Accordingly, derivatives of these compounds which produce a formulation having the desired antipathogenic and/or phytotoxic effect are considered equivalents of the invention. Depending on its structure, a given saponin can have a particular pesticidal property and lend use with the present formulations. Generally an effective amount of saponin is of the range 0.01 to 3% and most preferably about 0.25% v/v aqueous solution of 10° brix saponin extract.

Additional components such as an aqueous preparation of a salt of a polyprotic acid such as sodium bicarbonate, sodium sulfate, sodium phosphate or sodium biphosphate optionally can be included in the formulation, to increase the antifungal properties of the formulation. The resulting emulsion is diluted to an appropriate concentration for use. Other compounds which can be included in the composition include an antifreezing component such as glycerol,

propylene glycol, ethylene glycol and/or isopropyl alcohol, and a gum or gum-like material as xanthan gum, acacia gum, gelatin, hydroxypropyl methyl cellulose, as are described in U.S. Patent No. 5,290,557. If these compounds are to be used preharvest (*i.e.*, on a living plant) the formulation should be tested for phytoxicity and/or toxicity to the host plant prior to use.

The most effective formulations for compositions that include compounds of formula (1), (2), (3), (4) and/or (5) can be determined using protocols such as those described in the Examples and other methods known to those of skill in the art. Each formulation is evaluated for its effect on specific pests and/or plant host using any of the compounds of formula (1) as well as other components of the formulation such as Tween 80 and/or sodium bicarbonate, with the combination and effective amount of each component adapted for a particular application to minimize toxicity while maintaining or increasing the antipathogenic effect of the formulation. The effective amount of each component may be determined by systematically varying the amount of that component in a test formulation, treating a plant of interest with the test formulation, and monitoring the level of pest infestation compared to an untreated control plant. An effective amount of a test component is identified as the amount that controls the growth of the pathogen on the plant host, thus reducing the disease rating of the plant. The mean percentage disease resistance (MPDC) can be calculated for each particular application. MPDC is defined by the formula:

$$MPDC = \frac{(MDIC\text{-}MDIT)}{MDIC} \times 100$$

and

MDIC = Mean % of disease incidence in untreated controls
MDIT = Mean % of disease incidence in the treatment

Generally, for effective pathogen control the mean percentage of disease control (MPDC) is greater than 60%, preferably at least about 70%.

The formulations also are evaluated for phytotoxicity; at least one evaluation of the toxicity of the formulations is on living plants of the host variety. Phytotoxicity is rated as follows in order of increasing severity of toxicity: 0-plants without any symptoms; 1-very slightly browning of hypocotyl (no other symptoms); 2-some wilting of plant, dying of lower leaves, some browning of vascular system; 3-wilting of entire plant, leaves dying, hypocotyl with external and internal symptoms; 4-necrosis of stem, plant dying. It is preferable that the formulation used have a phytotoxicity rating of 2 or less, more preferably 1 or less. As an example, the effects of cinnamic aldehyde in a range from 0.1 ppm to 25,000 ppm on powdery mildew is evaluated. The mean disease control can be increased by using higher doses of cinnamic aldehyde, and/or adding other compounds of formula (1), or by increasing the substantiveness of the formulation by adding detergent, and the like.

An effective growth modulating amount of a test component is identified as the amount that decreases the extent to which a pest colonizes a host plant, either by killing the pest or preventing its reproduction. An effective growth modulating amount of one or more compounds of formula (1), (2), (3), (4). or (5) is generally about 0.01 g/l to 25 g/l, more preferably about 1 g/l to 20 g/l, and most preferably about 5 g/l to 10 g/l. In a preferred embodiment, the formulation includes an effective growth modulating amount of $\alpha$-hexyl cinnamic aldehyde, and/or cinnamic aldehyde and/or coniferyl aldehyde and/or a balsam in a formulation containing Tween 80 or saponin as an emulsifier and optionally sodium bicarbonate. A preferred formulation is an emulsion which contains $\alpha$-hexyl cinnamic aldehyde or cinnamic aldehyde (0.1% to 10% by weight), balsam (0.1 to 2% by weight), and may include the salt of an aprotic acid (8% to 12% by weight) and the balance water. Formulations with 6-12% of an aprotic acid are preferred. Generally, the total amount of aldehyde(s) present in the formulation is 10% or less. The preferred formulation for treating powdery mildew, rust and spores, as well as aphids is an emulsion which contains cinnamic aldehyde and/or coniferyl aldehyde (0.001% to 10% by weight), the salt of a polyprotic acid (4% to 12% by weight), an emulsifier (1% to 4% by weight), and the balance water. A synergistic amount of balsam is determined by methods described herein and known to those of skill in the art. The formulations are effective without the use of antioxidants other than the inherent antioxidant properties of particular aromatics. for example, coniferyl aldehyde.

Stability of the formulation can be evaluated by a variety of methods, including accelerated tests in which a formulation of interest is exposed to elevated temperatures over a set time. Samples of the formulations are taken at regular intervals and analyzed chemically by methods known to those skilled in the art to determine the rate and nature of degradation. For example, HCA can be analyzed by Gas Liquid Chromatography (GLC), using a 30 meter non-polar polydimethylsiloxane capillary column (e.g. HP-1, Hewlett-Packard, or SPB-1, Supelco) and a flame-ionization detector. Using helium as a carrier gas (8 ml/min.) and a column temperature of approximately 240°C. the (E)-cis isomer (major component) has a retention time of approximately 6.0 minutes and the (Z)-trans isomer (minor component) has a

retention time of approximately 6.3 minutes.

For applications where the formulation is used to prepare the ground or other growth substrate for planting of host plants susceptible to particular pathogens, to apply to an already infested growth substrate, or to harvested material the formulations of the subject invention can be added directly to the rhizosphere, the substrate or the harvested material. Alternatively, the aromatic compounds can be bound to a solid support or encapsulated in a time release material. Where a solid carrier is used, materials which can lead to oxidation of the active aromatics are avoided. Examples of delivery systems include starch-dextran, and the like. *See* Yuan *et al*., *Fundamental and Applied Toxicology* (1993) 20: 83-87, for examples of delivery systems. Also *see* Kawada *et al*. (1994) 10: 385-389.

In addition to the specific compounds of the formulas (1), (2), (3), (4) and (5) and optionally saponin as set forth above, derivatives of any of these compounds that produce a compound of the formula identified above upon action of a biological system on the derivative are considered to be equivalent to compounds of the invention. Thus application of precursor compounds to plant parts or tissues or harvested materials is equivalent to the practice of the present invention. Biological conversion of precursor compounds into aromatic aldehydes is described in, for example, U.S. Patent Application No. 5,149,715 and references cited therein. *See* also Casey and Dobb *Enzyme Microb. Techol*. (1992) 14: 739-747. Examples of precursor compounds include those in pathways relating to acquired and/or systemic resistance to plant resistance to plant pathogens such as those in the amino acid ammonia lyase pathways, and include phenylalanine (to produce cinnamic acid). Other precursor compounds of interest include those in the biological pathways for the production of lignins and coumarins, for example tyrosine to produce p-coumaric acid. Accordingly, precursors and derivatives of these compounds such as balsam components which produce a formulation having the desired antipathogenic effect are considered equivalents of the invention.

The method of the present invention is carried out by introducing into a target pathogenic organism a sufficient amount of an antipathogenic agent to impair growth and/or viability of the target pathogenic organism. A formulation containing the antipathogenic agent is introduced to a plant tissue or part either pre- or post-harvest. Methods of application include spraying, pouring, dipping, injecting, and the like, the active ingredient in the form of a concentrated liquid, solution, suspension, powder and the like. For example, the formulation is sprayed on as a wet or dry formulation to the surface and/or underside of the leaves or other plant tissue or part of a plant infected with a plant pathogen, or of a plant susceptible to infestation with a plant pathogen, preferably to the point of run off when a wet formulation is used. The plants can be sprayed prior to or after infestation, preferably prior to infestation. However, in order to minimize damage to the host plant, where feasible, it is preferable to treat older plants, as young green leaves tend to be more sensitive to phytotoxicity. A plant growth promotant, such as saponin, is optionally used pre-harvest either in the antipathogenic formulation or as a separate formulation. Alternately, the formulation can be applied wet or dry, either as part of an irrigation schedule or as a separate application, to the rhizosphere where it can contact the roots and associated pathogenic organisms which colonize the roots. In some instances, time-release formulations may find use, particularly for applications to the rhizosphere, or to post-harvest materials.

Depending upon the target organism, the aromatic aldehyde used can be microencapsulated in a polymer microcapsules containing the aromatic aldehyde. In an application of controlling a plant pest, the polymer shell material is preferably a biodegradable material, such as beeswax, carnauba wax, gelatin, sucrose, starch or dextran, so that the shell can be degraded to release the subject compounds to the target pest or its habitat. To encapsulate the subject compound in a polymer, a first prepolymer is dissolved in the core material of an aromatic aldehyde. The resulting solution is then dispersed in the continuous phase (usually water), which usually contains one or more dispersing agents. A second prepolymer is then added to the resulting emulsion. The shell wall forming reaction occurs at the oil/water interface of the emulsion droplets. The resulting suspension of microcapsules, which encapsulates the aromatic aldehyde can then be further formulated to produce the final product. For example, cinnamic aldehyde, coniferyl aldehyde or $\alpha$-hexyl cinnamic aldehyde can be microencapsulated at about one micron size in beeswax or carnauba wax and sprayed to plants susceptible for infestation by pests.

Alternatively, the aromatic aldehyde, can be coupled to a solid support, optionally through a linker such as a binding domain derived from a polysaccharidase, where the solid support is a polysaccharide such as cellulose, particularly microcrystalline cellulose. The preparation of cellulose binding domains is described in U.S. Patent Nos. 5,340,731; 5,202,247 and 5,166,317. Binding domains from scaffold proteins also can be used. *See* Shoseyev *et al;* PCT application PCT/0594/04132. The aromatics can be coupled to the binding domains or other solid support, with or without a cleavable bond, using methods known to those skilled in the art.

Solid or microencapsulated forms of the active ingredients are particularly useful for treating or preventing infestations of soil-borne pathogens. Analytical chemical techniques are used to determine and optimize rate of release of the active ingredient from the solid or microencapsulated form. For qualitative purposes, gas chromatography techniques can be used to determine the amount of aromatic released. For example, samples of encapsulated (pelletized) product are mixed with the soil types selected and sampled at different time periods to measure release. Volatile gases released from the formulation can also be analyzed. The activity and stability of foliar and drip irrigation applications of the formulations over time also can be evaluated by GC methodology using methods known to those skilled in the

art. Methanol or alcohol extractions of the formulations also can be prepared for HPLC analysis.

One or more components of the present formulations can be produced in the plant of interest by modulating the expression of one or more nucleotide sequences encoding one or more enzymes or an enzyme pathway or cluster required to control the level of the compound of interest in the plant, plant part, plant cell, specific plant tissue and/or associated with a particular stage of plant growth. The enzyme can be in a biosynthetic pathway or a degradation pathway and the regulation will be up or down, respectively, to modulate expression of either an endogenous plant gene or a transgene supplied exogenously to the plant. Down regulation can also be achieved using antisense nucleotide sequences. Expression is intended to include transcription alone when the nucleotide sequence is RNA. An indigenous plant gene is one which is native to the genome of the host plant. An endogenous plant gene is one that is present in the wild-type genome of the plant host of interest, and includes a gene that is present as a result of infection of the plant (for example, a viral gene) or otherwise naturally incorporated into the plant genome. The host plant also can be modified by recombinant means or by traditional plant breeding methods to introduce one or more genes exogenous to the host plant which encode enzymes that control the level of the compound of interest and as such are in the synthetic pathway for one or more aromatic compounds or compounds of formula (1), (2), (3), (4) or (5). By modulation of gene expression is intended control of production of a gene product of interest at the level of transcription, translation and/or post translation. The level of the compound of interest is controlled by modulating the expression of one or more endogenous genes or transgenes encoding one or more enzymes required to synthesize the compound of interest.

Methods for modulating gene expression in plants are known in the art. Variation in growth conditions or exogenous application of compounds to a plant can affect gene expression. For example, the formulations of the present invention can be used to induce systemic plant resistance through modulation of endogenous gene expression. At the molecular level, gene expression depends substantially on the transcription, translation and termination control regions which regulate expression of a structural gene coding region. By exploiting the plant signals which regulate these control regions or by the direct recombinant manipulation of the control regions, expression of a gene encoding an enzyme required to control the level of cinnamic aldehyde, for example. can be modulated. Where the transgene is exogenous to a plant host, the transgene includes control regions that are selected and designed to achieve the desired level and timing of gene expression. As appropriate, the control regions are homologous (native) or non-homologous (non-native) to the gene of interest. By "homologous" is meant that the control region(s) is from or substantially similar to a control region normally associated with the gene of interest. By "non-homologous" is meant that the control region(s) originates from a different nucleotide source or sequence or is substantially different from the control region(s) normally associated with the gene of interest. For example, if the enzyme coding sequence is non-homologous in source as compared to the control regions, in order to have expression of the gene in a plant cell of interest, transcriptional and translational initiation regulatory regions or promoters functional in these plant cells are provided operably linked to the coding sequence. Transcription and translation initiation signals functional in plant cells include those from genes which are indigenous or endogenous to a plant host or other plant species, and direct constitutive or selective expression in a plant host, and include sequences from viruses such as CaMV which infect plants.

Of particular interest are the gene control regions that selectively regulate structural gene expression in a plant, plant part, plant cell, specific plant tissue and/or associated with a particular stage of plant growth. Preferred are those control regions that are known in the art, and in particular, transcriptional control regions or promoters that can be used to modulate the expression of a gene encoding an enzyme required to control the level of a component of formula (1), (2), (3), (4), (5) and/or saponin in a plant, plant part, plant cell, or specific plant tissue and/or are associated with a particular stage of plant growth. For example, promoters providing for differential expression patterns in fruit are described in USPN 4,943,674 and USPN 5,175,095; seed in USPN 5,315,001; and in rapidly developing tissues and tender shoots in USPN 5,177,011.

A preferred method for producing a desired component of the present formulations in a plant host is through recombinant means, particularly by modifying the level of at least one compound of interest of the formula (1), (2), (3), (4), (5) and/or saponin in plant tissues of interest through construction of transgenic plants using recombinant techniques known in the art. The methods involve transforming a plant cell of interest with an expression cassette functional in a plant cell comprising as operably linked components in the 5' to 3' direction of transcription, a transcriptional and translational initiation regulatory region, joined in reading frame 5' to a DNA sequence encoding one or more enzymes capable of modulating the production and/or required to produce the compound of interest, and translational and transcriptional termination regions. Expression of an enzyme required to produce the compound of interest provides for an increase in production of the compound as a result of altered concentrations of the enzymes involved in the compounds' biosynthesis. Of particular interest is the selective control of saponin, cinnamic and/or coniferyl aldehyde and/or cinnamic acid production in plant tissues such as leaves, roots, fruits and seeds.

For cinnamic aldehyde biosynthesis in a tissue of interest, plant cells are transformed with an expression cassette comprising DNA encoding a structural gene for one or more enzymes required to synthesize cinnamic aldehyde and capable of increasing the amount of cinnamic aldehyde in the tissue of interest. Similarly, for selective control

of saponin biosynthesis in a tissue of interest, plant cells are transformed with an expression cassette comprising DNA encoding a structural gene for one or more enzymes required to synthesize saponin and capable of increasing the amount of these compounds in the tissue of interest. Of particular interest are those genes encoding one or more enzymes capable of metabolizing a precursor compound required for the biosynthesis of the saponin, cinnamic and/or coniferyl aldehyde compound of interest from substrates normally found in a plant cell. More particularly of interest is the transgenic expression of at least one compound of the formula (1), (2), (3), (4), (5) and a saponin.

DNA constructs for expressing a gene of interest are prepared which provide for integration of the expression cassette into the genome of a plant host. Integration can be accomplished using transformation systems known in the art such as *Agrobacterium*, electroporation or high-velocity microparticle-mediated transformation. Depending upon the application, saponin or one of the other compounds of interest can be preferentially expressed in a tissue of interest and/or a particular organelle. Tissue specificity is accomplished by the use of transcriptional regulatory regions having the desired expression profile. Translocation of the enzyme to a particular organelle is accomplished by the use of an appropriate translocation peptide. Methods for tissue and organelle specific expression of DNA constructs have been described and are known in the art.

To verify regulation and expression of the gene of interest, various techniques exist for determining whether the desired DNA sequences present in the plant cell are integrated into the genome and are being transcribed. Techniques such as the Northern blot can be employed for detecting messenger RNA which codes for the desired enzyme. Expression can further be detected by assaying for enzyme activity or immunoassay for the protein product. Most preferably the level of the compound of interest present in a plant host is measured using methods known in the art. A desired phenotype, for example, is increased saponin and/or aromatic aldehyde or acid content in a plant tissue of interest as measured by expression of the gene of interest and/or the level of saponin or aromatic compound present in the plant host as compared to a control plant.

For introduction of one or more compounds of the present formulations to the target organism, a plant host expressing a gene encoding an enzyme required to control the level of the compound of interest results in the exposure of a target organism to at least one component of the antipathogenic formulation. In another embodiment, selective expression of the gene of interest induces systemic plant host resistance to pathogen attack or colonization. At least one component of the antipathogenic formulation can be expressed by the plant host and optionally other components of the antipathogenic formulation are exogenously applied to the plant host so that the combination elicits the desired antipathogenic effect when either directly or indirectly introduced to the target organism. Transgenic plants having an increased ability to accumulate aromatic compounds such as cinnamic acid, cinnamaldehyde, $\alpha$-hexyl cinnamic aldehyde and coniferyl aldehyde, in addition to autoprotection against plant pathogens can be used as a source of aromatic compounds for extraction and subsequent use as a chemical pesticide.

Accumulation of aromatic compounds can be achieved by downregulating the expression of specific plant genes that encode enzymes which either cause further metabolism of the desired aldehydes or divert metabolic intermediates away from the desired aldehydes. In the case of cinnamaldehyde, for example, this involves downregulating the expression of cinnamate 4-hydroxylase (CA4H) and cinnamic alcohol dehydrogenase (CAD). CA4H ordinarily diverts some cinnamic acid away from cinnamaldehyde to produce *p*-coumaric acid, itself a metabolic intermediate. Reducing CA4H activity alone generally is not sufficient to cause accumulation of cinnamaldehyde because CAD can rapidly convert cinnamaldehyde to cinnamyl alcohol, which then becomes incorporated into lignin or accumulates as glycosides. Simultaneously reducing both CA4H and CAD activities results in increased metabolic flux from cinnamic acid into cinnamaldehyde and decreased conversion of cinnamaldehyde into cinnamyl alcohol. Some cinnamaldehyde becomes incorporated into lignin but cinnamaldehyde (either free or as glycosides) also accumulates to above-normal levels, particularly at times when the biosynthesis of cinnamic acid is elevated. This occurs when the level of phenylalanine ammonia lyase (PAL; the first and rate-limiting step in general phenylpropanoid metabolism, Hahlbrock and Scheel, (1989) *Annu. Rev. Plant Physiol. Plant Mol. Biol.* 40:347-369) activity is high, a situation that naturally occurs in plants in response to a wide range of stimuli including invasion by fungal pathogens and mechanical damage associated with wounding and insect feeding.

A number of plant CA4H and CAD genes have been cloned and their sequences are available from GenBank. Portions of these genes that include nucleotide sequences that are conserved among different plant species can be used directly in a plant expression vector (antisense or sense orientation) to suppress the expression of the corresponding endogenous genes (e.g., Pear *et al*. (1993) *Antisense Res. and Develop.* 3:181-190, Napoli *et al*. (1990) *The Plant Cell* 2:279-289. *See* also USPN 5,107,065). More preferably, these conserved gene sequences are used to isolate CA4H and CAD cDNA clones from a cDNA library of the plant species that is to be modified. The resulting cDNA clones, or portions thereof, are then introduced into a plant expression vector (antisense or sense) and used to transform the plant(s) of interest. DNA constructs according to the invention preferably comprise a sequence of at least 50 bases which is homologous to the endogenous CA4H or CAD genes.

A recombinant DNA molecule is produced by operatively linking a vector to a useful DNA segment to form a plasmid that can be used for plant transformation. A vector capable of directing the expression of RNA from a cloned

portion of a gene is referred to herein as an "expression vector." Such expression vectors contain expression control elements including a promoter. Typical vectors useful for expression of genes in higher plants are well known in the art and include vectors derived from the Ti plasmid of *Agrobacterium tumefaciens* described by Rogers *et al., Methods in Enzymology* (1987) 153:253-277. A common promoter that is used to provide strong constitutive expression of an introduced gene is the cauliflower mosaic virus (CaMV) 35S promoter (available from Pharmacia, Piscataway, NJ). Either constitutive promoters (such as CaMV 35S) or inducible or developmentally regulated promoters (such as the promoter from a PAL gene or the endogenous CA4H or CAD genes) can be used. Use of a constitutive promoter tends to affect functions in all parts of the plant, while use of an inducible or developmentally regulated promoter has the advantage that the antisense or sense RNA is produced substantially only in a desired tissue and under the conditions it is required. The use of developmentally regulated promoters is preferred because the down-regulation of phenylpropanoid biosynthesis is known to be capable of producing undesirable side-effects on the development of transgenic plants containing a heterologous PAL gene (Elkind *et al.* (1990) *Proc. Nat. Acad. Sci.* 87:9057-9061.

A number of different transformation methods are available for the routine transformation of a wide range of plant species. One method that is particularly efficient for the transfer of DNA into dicotyledonous plants involves the use of *Agrobacterium*. In this method the gene of interest is inserted between the borders of the T-DNA region that have been spliced into a small recombinant plasmid with a selectable marker gene (for example encoding neomycin phosphotransferase II or phosphinothricin acetyltransferase). The recombinant plasmid is then introduced into an *Agrobacterium* host by transformation or triparental mating. The *Agrobacterium* strain carrying the gene(s) of interest is then used to transform plant tissue by cocultunng the bacteria with an appropriate plant tissue (*e.g.,* leaf disc). Transformed cells are selected in tissue culture using the appropriate selection agent and plants are then regenerated *(see* Horsch *et al.* (1985) *Science* 227:1229-1231. Other methods that have been used in the transformation of plant cells, and in particular the more recalcitrant crop plants, include biolistics and electroporation (for detailed protocols, *see Sanford et al.* (1993) *Methods in Enzymology* 217:483-509; and Potter (1993) *Methods in Enzymology* 217:461-478.

Once transgenic plants have been produced, conventional enzyme assays for CA4H and CAD are used to determine the level of suppression of enzyme activity achieved in different transformants. It is likely that only a small fraction of the transformants produced will have a sufficiently low residual enzyme activity to cause the accumulation of aromatic aldehydes without also producing some undesirable side effects on plant development. For this reason, a preferred method of producing the desired transformants with both CA4H and CAD suppressed is to introduce the two genes separately into different transformants and then combine them by standard sexual crosses. This permits a larger number of combinations of level of gene suppression to be evaluated at the same time.

An alternative to overproducing aromatic compounds in transgenic plants is to use the plant genes to confer on a microbial host the capability of synthesizing specific aromatic compounds and/or saponins. The resulting transformed microorganisms can be used either to produce the aromatic compounds in a fermentation system or as a natural delivery system of the aromatic compounds in viable or non-viable microbial preparations. Yeasts, especially *Saccharomyces cerevisiae*, are preferred organisms for this purpose because they already have been engineered for high-level expression of phenylalanine ammonia lyase (Faulkener *et al*. (1994) *Gene* 143:13020) and a plant cinnamate 4-hydroxylase has been shown to function in yeast (Urban *et al*. (1994) *Eur. J. Biochem*. 222:843-850.

The expression of phenylalanine ammonia lyase introduces the capability to produce cinammic acid from phenylalanine. Two additional enzymic steps are required to produce cinnamaldehyde from phenylalanine. In plants, these steps are catalyzed by the enzymes cinnamate:CoA ligase (CL) and cinnamoyl CoA reductase (CCoAR) but as 4-coumarate CoA ligase (4CL) can also use cinnamic acid as substrate (Knobloch, and Hahlbrock (1977) *Arch. Biochem. Biophys*. 184:237-248), 4CL can be used instead of CL. More than 20 cloned PAL genes and more than 6 4CL genes have been described in sufficient detail (GenBank) to facilitate their use in practicing the current invention. A gene for a CCoAR is obtained by applying standard gene cloning techniques to isolate a cDNA clone using as a probe sequence derived from the amino acid sequence of the N-terminus, or peptide fragments, of the purified protein. CCoAR has been purified and partially characterized from soybean cultures (Wengenmayer *et al.* (1976) *Eur. J. Biochem*., 65: 529-536; Luderitz and Grisebach (1981) *Eur. J. Biochem.* 119:115-124), spruce cambial sap (Luderitz and Grisebach, *supra),* poplar xylem (Sarni *et al*. (1984) *Eur. J. Biochem*. 139:259-265) and differentiating xylem of *Eucalyptus gunnii* (Goffner *et al.* (1994) *Plant Physiol.* 106:625-632). The preferred method of purification is that of Goffner *et al,* (*supra*) because it results in a single protein band on SDS-polyacrylamide gels that an be used for protein sequencing. For the expression of α-hexyl cinnamic aldehyde, the gene that codes for the enzyme that catalyzes the addition of the α-hexyl group to cinnamic aldehyde also is inserted into the microbial host or plant. The gene can be cloned, for example, from rice plants.

The cloned genes are introduced into standard expression vectors and used to transform a microbial host, preferably yeast, by standard transformation techniques such as electroporation (Becker and Guarante (1991) *Methods in Enzymol*. 194:182-187). Standard enzyme assays are used to confirm the functional expression of the engineered genes and assays for aromatic aldehydes are used to select strains with maximal production. Because aromatic compounds have antimicrobial properties it is preferred to use expression vectors that cause expression of the introduced

genes only late in the growth cycle or in response to a chemical inducer. It may also be desirable to grow the engineered microbial host in an immobilized whole cell reactor (*e.g*., Evans *et al*. (1987) *Biotechnology and Bioengineering* 30: 1067-1072) to prevent the aromatic compounds from accumulating in the culture medium.

The subject formulations and methods are useful for treatment of plants that are colonized by pathogenic organisms. These include flowering plants, grasses, including ornamental turf grass, bent grass, vegetables, cereals and fruits including tomato, potato, artichoke, strawberries, corn, cereal grains, onion, cucumber, lettuce, tobacco, and citrus such as orange, lemons, limes and grapefruit, as well as bell peppers and grapes, and fruit trees such as peach, apple and cherry, ornamentals such as roses and trees, particularly conifers. Also included are crops intended for consumption by fish, fowl and animals, including humans, directly or indirectly. By "directly or indirectly" is intended that the crops could be ingested, for example, by humans (direct consumption), or that it is the nonhuman animal or fowl which ingests the crop and is in turn ingested by humans (indirect consumption). Crops intended for consumption include tobacco, fish, animal and fowl fodder, crops intended for processing into alcohol or food products such as corn syrup, and the like.

Target pathogenic organisms include fungi that colonize a plant or plant part, particularly a surface of a plant part. The optimal time and method for applying the formulations is determined by the particular part of the plant colonized by a fungus and the time in the plant life cycle at which a particular infestation occurs or is likely to occur. For example, to treat powdery mildew, rust and other pathogens which colonize the leaves of the host plant, the host plants are sprayed to run off with a formulation of the invention. The amount of compound(s) of formula ( I ) used will vary depending in part upon the target pathogen and the host plant and can be determined empirically by evaluating the sensitivity of the target organism to the formulation and the phytotoxic effects of that formulation or the host plant. The plants can be sprayed prior to or after infestation, preferably prior to infestation. However, in order to minimize damage to the host plant, where feasible, it is preferable to treat older plants, as young green leaves tend to be more sensitive to phytotoxicity. Alternatively, transgenic crops can be used, which express one or more components of the formulation in an amount sufficient to inhibit growth of the pathogen and/or kill the pathogen. Preferably the component(s) is expressed in the tissue colonized by the pathogen, for example the leaves. When it is desired to treat the roots or meristems of a plant, in some instances, it can be advantageous to apply the formulation at a time of day and/or season and/or growth cycle of the plant when translocation is from the leaves to the roots, or other plant tissue of interest.

Of particular interest is treatment of plants affected by powdery mildew which is caused by target organisms the fungal family *Erysiphaceae.* For example, the following species cause powdery mildew in the indicated plants: *Erysiphe cichoracearum*: begonia, chrysanthemum, cosmos, cucurbits, dahlia, flax. lettuce and zinnia; *E. graminis*: cereals and grasses; *E. polgoni*: beans, soybeans, clovers, and other legumes, beets, cabbage and other crucifers, cucumber and cantaloupe, delphinium and hydrangea; *Microsphaera alni*: blueberry, catalpa, elm, lilac, oak, rhododendron, and sweet pea; *Phyllactinia sp.:* catalpa, elm, maple and oak; *Podosphaera leucotricha*: apple, pear and quince; P. *oxyacanthae*: apricot, cherry, peach and plum; *Spaelrotheca macularis*: strawberries; S. *mors-uvae*: gooseberry and currant; *S. pannosa*: peach and rose; and *Uncinula necator*: grape, horse chestnut and linden.

Also of particular interest is the treatment of plants affected by rust caused by *Basidiomycetes,* particularly of the order *Uredinales.* There are about 4,000 species of rust fungi. The most important rust fungi and affected plants include: *Puccinia:* rust of numerous hosts such as wheat and all other small grains (*P. graminis*); yellow or stripe rust: wheat, barley and rye (*P. strliformis*); leaf or brown rust: wheat and rye (P. *recondita);* leaf or brown dwarf rust of barley (*P. hordei*); crown rust of oats (*P. coronata*); corn rust (*P. sorghi*); southern or tropical corn rust (*P. polysora*) sorghum rust (*P. purpurea*); and sugarcane rusts (*P. sacchari* and *P. kuehnii*). *Puccinia* also causes severe rust diseases on field crops such as cotton (P. stakmanii), vegetables such as asparagus (*P. asparagi)*, and flowers such as chrysanthemum (P. *chrysanthemi*), hollyhock (*P. malvacearum*), and snapdragon (*P*. *antirrhini*). *Gymnosporangium*, causes the important cedar-apple rust (*G. juniperi-virginianae*) and hawthorn-cedar rust (*G. globosum*). *Hemileia,* causes the devastating coffee leaf rust. (*H. vastatrix*). *Phragmidium,* causes rust on roses and yellow rust on raspberry.

Other fungal species that cause rusts and are treatable using the subject formulations include *Uromyces:* legumes (bean, broad bean, and pea) (several *Uromyces* species) and carnation (*U*. *caryophyllinus*); *Cronartium*: severe rusts of pines, oaks, and other hosts, such as the white pine blister rust (*C. ribicola);* fusiform rust of pines and oaks (*C. quercuum f.* sp. *fusiforme*); eastern gall or pine-oak rust (*C*. *quercuum* f. sp. *virginianae*); pine-sweet fern blister rust (*C. comptoniae);* pine-Comandra rust (*C. comandrae*); and southern cone rust (*C*. *strobilinum*). Others include *Melampsora,* which causes rust of flax (*M. lini*); *Coleosporium*, which causes blister rust of pine needles (*C. asterinum*); *Gymnoconia,* which causes orange rust of blackberry and raspberry; *Phakopsora,* which causes the potentially catastrophic soybean rust (*P. pahyrhizi*); and *Tranzschelia*, which causes rust of peach.

The subject formulations and methods also are useful to prevent and treat infestation by organisms that colonize other parts of plants, such as the roots and fruit. For fungi that affect plant roots, such as *Fusarium* sp., *Aspergillus,* and *Verticulum* (including *V. alboatrium* and *V. dahlise),* infestations are preferably treated by foliar application of the subject formulations, with the active ingredient reaching the roots by translocation or by induction of a systemic acquired resistance (SAR). To treat fruit infestations, such as black spot, the subject formulations are applied during and after

fruit development, preferably directly to the developing fruit. Other target fungal organisms include *Phragmidium spt*; *Diplocaopan rosae*; *Sphaerotheca tannosa*; *Oibiapsis sicula; Phytophoya taraesitica; Puccinia spp*; *Alternaria sp; Susaiun spp; Botrytis cinera; Sclerotinia Homoeocarca;* Dutch Elm disease (*Ceratocystis ulmi*) and oak wilt (*C. fagacearum*). Also included are blue-green algae (Cyanobacteria).

The subject formulations also are useful against insects, particularly those of the orders *Orthoptera*; *Thysanoptera* which includes thrips; and *Homoptera* which include aphids, leafhoppers, white flies, mealy bugs, cicadas and scale insects. It is a theory of the invention that the insects which are susceptible to treatment with the subject formulations are those which harbor symbiotic bacteria in their gut. Accordingly, insects other than those listed which harbor symbiotic material also can be controlled with the subject formulations. Other target organisms include arachnids, particularly spider mites (*Arthropoda*). Additional insect targets include those of the orders *Coleoptera* such as corn root worm and cotton boll weevil; and *Lepodoptera,* which includes codling moth.

Of particular interest is treatment of phylloxera infestation in grapes. The root form of grape phylloxera (*Daktulosphaira vitifoliae* Fitch) is a devastating grape pathogen. Plant damage is not due solely to phylloxera feeding but rather is substantially due to invasion of secondary fungi, organisms which are not controlled by insecticides. Typically, phylloxera are found as deep as the roots of the host plant, which may be eight feet or deeper. Thus, for treatment of phylloxera infestation in grapes, the formulation is delivered to the plant roots directly, such as by injection of liquid or solid formulation into the soil surrounding the roots. Alternatively, the formulations can be applied to the foliage or other accessible part of the plant and translocated to the root area through the plant vascular system or by induction of an SAR. As in the case of treatment of powdery mildew and rust, transgenic crops can be used for treatment and/or prevention of phylloxera; the preferred tissue of expression of components of formula (1) is the root.

Compounds of formula (1) can be used for treatment of phylloxera at different stages of growth. The compounds can be formulated or differentially applied to target particular stages of phylloxera growth such as the egg, nymph and adult stages. Efficacy of a particular formulation and treatment protocol can be evaluated by any number of means, such as assessing phylloxera mortality, feeding, and/or vacated feeding sites after treatment under field and/or laboratory conditions, or by improved health of the infested vines. In addition, efficacy of a particular formulation and treatment protocol can be evaluated through analysis of vine physiology in metabolizing, translocating and/or reacting to the formulation treatment by methods known in the art. Factors other than the normally tested dosage, formulation and timing of treatments also are considered to determine involvment of phylloxera phenology, plant phenology, plant health, presence of a graft union and cultivar (both of the scion and rootstock).

The subject methods and formulations also are useful for treating infestations of turfgrasses. Certain biotic agents of noninfectious diseases damage turfgrasses by competition. Algae frequently damage turfgrass by taking over and occupying the void that remains after turf is thinned by infectious disease. Blue-green grass algae (species of *Ajanobacteria*) develop as a black scum on the surface of overly wet soils. The algae reduce exchange of gases between air and soil and also may introduce chlorosis in plants. Black-layer is a physical condition that is primarily associated with putting greens on golf courses. It is noted especially in turfs with a high sand content. In addition, turfgrass fungal diseases can be treated using the subject formulations. *Sclerotinia* dollarspot, *Pythium* blight and *Rhizoctonia* blight are devastating diseases of various turfgrass species, especially bent grasses. The efficacy of the formulations for treatment of turgrasses can be determined by varying the components of the formulation, the application regime, volume of application and/or the rate of application. The evaluation can be conducted under field and/or laboratory conditions, with consideration given to environmental conditions such as humidity, temperature, light quality, quantity and intensity, soil type and fertility, moisture content, drainage and the like. The effect of formulation persistence and activity can be determined also by comparison of acropetal translocation and persistence with daily mowing and removal of the grass blades. The formulations may be applied at variable application rates and concentrations, and application regimes to obtain a desired level of disease control and phytotoxicity. Compositions of formula (2) are preferred for this application, with formulae (3), (4) and (5) being more preferred.

Of particular interest is use of the subject formulations to control *Sclerotinia* dollar spot (*Sclerotinia homeocarpa*). *Sclerotinia* dollar spot is a widespread, persistent and costly disease that occurs on most turfgrass species throughout the world. Environmental conditions that favor dollar spot include long periods with high humidity and excess moisture. Low nitrogen fertility coupled with these environmental conditions increase the incidence of dollar spot disease. Fine-leafed grasses that form a dense and compact growth habit are especially at risk, making dollar spot a major threat to intensively managed golf course greens. Also of particular interest is use of the formulations to control diseases caused by *Pythium* species. Diseases caused by *Pythium* species are often referred to as *Pythium* blight, grease spot, spot blight, crown rot and root rot. *Pythium* also causes seedling disease. All turf grasses are susceptible to attack by *Pythium* spp., including creeping bentgrass, the most popular grass for golf course greens, and the new types of bentgrass grown in the southern United States. Resistant biotypes of *Pythium* typically do not respond to traditional fungicide application. The subject formulations typically are applied as a foliar spray prior to or upon the first appearance of conditions that promote growth of *Pythium* species. For *Pythium* blight, this is particularly when the weather is hot and humid and the nights stay warm.

Another preferred use of the methods and compositions of the invention is to control *Rhizoctonia* blight, commonly referred to as brown patch. *Rhizoctonia* blight is an especially rapid and destructive disease when environmental conditions favor its growth. Thus the formulations to be used generally should be applied when there is hot, humid weather, high nitrogen fertilization rates, dense foliar growth and/or frequent watering. *Rhizoctonia* blight control on turfgrasses is difficult and the traditional fungicides often fail for this disease. Control of *Rhizoctonia* blight of St. Augustine grass using the subject formulations also is of interest.

Of particular interest is the treatment and prevention of thrip, powdery mildew, black spot, botrytis and melon aphid infestation of oranamental flowers and other plants targeted by these pests. The subject formulations are of particular use for preventing or treating infestations of rose, christmas trees (*e.g.*, pine), and fruit trees and fruit. For example, the formulations are useful for treating peach against canker and apple against codling moth infestation, and grape from infestation by leaf roller, phylloxera, leaf hopper, botrytis, thrips, and powdery mildew. Preferred formulations are from the aromatic aromatic aldehydes of formulae (2) and (5), with formulae (3), (4) and (5) preferred. An example is the treatment of melon aphid infestation in cotton. The most important species is the cotton or melon aphid (*Aphis gossypii Glover*). Almost as soon as cotton has put out leaves, small, soft-bodied, pale-green plant lice fly to them and start to reproduce. Therefore, the subject formulations should be applied to cotton at an early stage in its development. Foliar spraying is preferred because the aphids feed above ground. The most important time for treatment is immediately prior to and/or during cool, wet seasons, when aphids can become sufficiently abundant to stunt and deform the plants which they colorize.

The subject invention also is useful for treating and preventing late blight. Late blight affects tomato, potato, eggplant and other potato family plants and results from infestation by *Phytophthora infestans*. The subject formulations are preferably applied to the plants during moist periods of mild temperature, which is when pathogenesis generally begins by settling of spores on plant surfaces. Likewise, control of boll weevil (*Anthonomus grandis Boheman*) is also of interest. The injury is caused by the adult weevils and their young or grubs. The adults puncture the squares and bolls by chewing into them. Application of the subject formulation should in particular be directed to this portion of the plant.

The treatment formulations are also useful for controlling the time of pollination of flowering plants. For example, to prevent or delay pollination the formulations are applied in an amount sufficient to repel bees and other pollinating insects. By adjusting the residuality of the formulation, one can control the length of time during which pollination is inhibited. On the other hand, for plants in which cross-pollination is required for fertilization, application of the formulation during this period should be avoided if the pollinating insect is repelled or killed by the formulation. In particular, species of *Vespidae*, including social wasps, paper-nest wasps, hornets and yellow jackets, are sensitive to the subject formulations.

Treatment of Dermaptera (European Earwigs (*Forticula aureculatia*)) with compounds of the invention also is of interest. Earwigs have extremely wide food tastes and feed on many diverse types of plant material, including flowers, vegetables and fruits, both pre- and post-harvest. Flower blossoms, corn silks (kernels) and new vegetable seedlings are particularly vulnerable. Therefore, the subject formulations are applied, for example, by foliar spraying to the growing plants and/or harvested plant parts sought after by the earwings.

Of particular interest is the postharvest treatment of cut flowers using the formulations of the subject invention to control growth of microorganisms, such as those found in vase solutions. The formulations also can increase the lifespan of cut flowers, which depends in part on control of vase solution microorganisms, such as bacteria and fungi, whenever the stems are in water. Treatment of cut flowers can be done by any number of means suited for a particular purpose, such as dipping the cut stem into a subject formulation of the invention and/or addition of the formulation to a vase solution or by dunking the entire cut flower into the formulation. In a preferred embodiment, postharvest control targets *Botrytis* blossom blight caused by *Botrytis cinerea* Pers: Fr. on cut flowers. *Botrytis* blossom blight is a widespread and destructive disease on greenhouse-grown roses and many other cut flower crops as well as grapes. Post harvest treatments with a test formulae are used to reduce gray mold caused by *B. cinerea*, delay rot on flowers (or fruit such as raspberries, strawberries, apples, pears) caused by *B. cinerea, Rhizopus*, and *Penicillium expansum.* Compounds of formulas (2) and (5) are preferred. More preferred is a formulation including cinnamaldehyde and/or $\alpha$-hexyl cinnamaldehyde in a formulation containing saponin and/or Tween 80. Efficacy of the formulations for promoting or preserving decapitated flower life can be evaluted by monitoring wilting of flowers, leaves, or stem, rehydration, bacterial growth and stem contamination, and postharvest control of microbial and physiological plugging.

The subject compounds of the invention can be used to target the three life stages of codling moth (*Cydia pomonella*), adults, eggs and neonate larvae, before they enter the fruit. For this application, it is useful to develop a susceptibility profile of these life stages to optimize the timing of application of the subject formulations. This is done by testing the susceptibility of eggs, neonate larvae, and adults to the formulations, both freshly applied and the residue that remains on a plant or plant part after the application.

The subject aromatic compositions also are useful for control of San José scale, which is an oddly shaped and immobile insect. Like mealy bugs, scales resemble disease organisms more closely than animals. There are two

families of scales: soft scales (*Coccidea*) which tend to feed on garden crops, and the armored scales *(Diaspididae)* that prefer orchard crops. Members of the superfamily *Coccidea,* they attach themselves to leaves, fruit and bark of many different plants. Therefore, the subject formulations are preferably applied to the leaves, fruit and bark of susceptible plants preferably before, but also after infestation.

The subject methods and compositions also are useful for control of mealybugs, which are similar to aphids, psyllids, and phylloxera. Mealybugs suck the juices from plants and spread disease, and the honeydew they excrete invites the growth of a sooty fungus which interferes with plant photosynthesis. Foliar application is used to control the sooty fungus, and the amount used preferably is an amount sufficient to induce SAR in the plant so as to control growth of the sap sucking insects in remote regions of the plant where the insects feed.

In addition to treating a host plant, seeds also can be treated using the subject formulations. The seeds can be dusted with a powder preparation (*see* U.S. Patent Application No. 4,978,686 for examples of inorganic materials to which the formulations can be adsorbed) or admixed in a plant substrate such as vermiculite. Seeds also can be obtained from transgenic crops, wherein the components of formula (1) have been produced in seed, preferably preferentially in seed. Seedlings grown under sterile conditions from treated seeds are free of susceptible fungi and insects. Additionally, seedlings also can be treated with the subject formulations. In some instances it may be necessary to adjust the treatment formulation so as to reduce any phytotoxicity associated with the treatment as tender young shoots are more likely to exhibit phytotoxicity symptoms from treatment.

The following examples are offered by way of illustration and not by way of limitation.

## EXAMPLES

### Materials and Methods

The chemicals used in the examples given below were obtained from the following sources: cinnamic aldehyde, Spectrum Chemical Company, N.J.; coniferyl aldehyde, APIN Chemical, U.K.; Tween 80 and sodium bicarbonate Spectrum Chemical Company, Gardena, C.A.; $\alpha$-hexyl cinnamic aldehyde, Firmenich, Plainsboro, New Jersey; saponin, Danco Corp., Fresno, C.A.

### Example 1

### Treatment of Powdery Mildew on Rose Cultivars

Potted Roses. Eight cultivars of rose were tested to investigate the effect of a cinnamic aldehyde/$NaHCO_3$ formulation on rust and spores. The cultivars used included Moss unnamed (Moss), Galica, Rosette Delize (Hybrid Tea), Rosa Rugosa Rubra (Rugosa), Abel Morrison (Hybrid perpetual), John Laing, Betty Prior, and Rose de Roi. Five (5) potted cultivars (Moss, Galica, Hybrid Tea, Rugosa, and Hybrid perpetual) were selected and assigned a disease rating after Paulus and Nelson (*supra*) for powdery mildew, rust and spores.[1] The Moss and Galica cultivars were 5 on a scale of 0-5 (where 0=no powdery mildew rust/spores lesions, 1=1-25, 2=26-50, 3=51-75, 4=76-90, and 5=>90% total leaves per bush). The Hybrid Tea and Hybrid perpetual were rated 3 and the Rugosa was rated 1. The Moss and Galica also were infected with rust equivalent to a 3 rating.

Each cultivar received a foliar spray of about 100 ml of a cinnamic aldehyde formula containing 5 g cinnamic aldehyde, 80 g $NaHCO_3$, 10g of Tween 80 and water to 1000g. In addition, 250 ml of 0.01% (v/v) aqueous solution of 10° brix saponin extract from the yucca shidigera plant was administered to each potted plant once a week beginning with the date of the first cinnamic aldehyde/$NaHCO_3$ treatment. Control plants received no treatment. A single treatment was eradicative of powdery mildew, rust, and spores through the final weekly field observation eight weeks later as compared to the no treatment controls which remained at disease ratings of 5, 3, and 4 for powdery mildew, rust and spores, respectively. Moreover, the treatment appeared to have induced systemic resistance. No phytoxicity was observed.

Field grown roses. Another experiment was designed for field grown cut flower rose to evaluate the efficacy of powdery mildew control by cinnamic aldehyde/$NaHCO_3$ during the same period (season) and environmental conditions. Powdery mildew and rust inoculum were high in the test field, and no additional inoculum was necessary to provide disease pressure. Cultivars John Laing, Betty Prior, and Rose de Roi were used in this investigation. Eight John Laing plants from a block row of sixteen were selected for treatment. Every other plant beginning with the first plant in the row was treated. Three Betty Prior plants selected from a block of six were similarly treated, as were two Rose de Roi plants from a block of four. A single foliar spray treatment (about 100ml) of a formulation containing cinnamic aldehyde (5g), Tween 80 (10g), $NaHCO_3$ (80g) and water to 1000g was applied to each setting of cultivars. Plants were an average of 0.86 m apart. The disease rating was the same as that used to evaluate powdery mildew in containerized cultivars. Controls were untreated plants. Absence of wind and exact spraying protected controls from

spray drift. The John Laing cultivars were young, 45-day-ol plants with a rating of 5 for powdery mildew. The Betty Prior cultivars were older (≥240 days), previously sprayed with Eagle (120 days prior) with a rating of 3 for powdery mildew and the Rose de Roi were 240-day-old plants with a rating of 2 for powdery mildew and 2 for rust using the same scale as provided above. Induced systemic resistance was determined by observing the number of lesions of powdery mildew and rust produced on each plant after treatment as compared to untreated controls. Weekly reviews were made of the various plants. The effect of the treatment regimen on growth rate was determined at the last field observation of each plant.

With the exception of the untreated controls and three plants of cultivar Betty Prior which had reinfection of powdery mildew with a rating of 3, all plants were free of powdery mildew at [1]Spores were evaluated only on Moss. Galica and the control plants. the end of the five week trial. No phytotoxicity was observed. All plants had new growth exceeding that of the untreated controls.

The Mean Percentage of Disease Control (MPDC) was calculated for every group of plants. The results were as follows for powdery mildew: John Laing, 98.3%; Betty Prior, 64.3%; Rose de Roi, 100%. The average for all three roses was 90.7% for powdery mildew. Rust was evaluated only on Rose de Roi, and was 85.0%. Effective fungicides for powdery mildew should provide a MPDC of ≥70% under greenhouse or field conditions, and for rust ≥65%.

Potted roses or field grown roses sprayed to runoff with an emulsion containing cinnamic aldehyde and sodium bicarbonate and concomitantly sprayed with saponin remained free of powdery mildew and rust for up to 56 days, while plants sprayed only with water did not. The treated plants also remained free of aphids. It has been reported that induced systemic resistance to powdery mildew of roses sprayed with Rubigon averages about 20 days. Mean disease control determinations of approximately 70% were obtained for roses sprayed with an aqueous solution of cinnamic aldehyde and coniferyl aldehyde or emulsions containing sodium bicarbonate and cinnamic aldehyde and/or coniferyl aldehyde. In parallel experiments, Benomyl gave a mean disease control of approximately 80%.

Example 2

Treatment of Fungi and Insects on

Roses with Coniferyl Aldehyde

Six cultivars of infected rose in dedicated experimental rose gardens were used. Four Mrs. John Laing (Hybrid perpetual) and two Marchionese of Londonderry (Hybrid perpetual) rose plants were treated with one of two formulations of coniferyl aldehyde. The low dose treatment (T1) was a formulation containing coniferyl aldehyde (5g), Tween 80 (10g), $NaHCO_3$ (80g) and 905g of $H_2O$ for 1000g of product. The high dose treatment (T2) was a coniferyl aldehyde formula comprising of 100g of coniferyl aldehyde, 20g Tween 80, 120g $NaHCO_3$, and 760g $H_2O$ for 1000g of product. *See* Table 1.

The first two Mrs. John Laing plants (P1 and P2) were assigned a disease rating of 3 for powdery mildew and rust using the rating system of Paulus and Nelson (*supra*). Mrs. John Laing plants 3 and 4 (P3 and P4) were assigned a disease rating of 4 and 5 respectively for powdery mildew and rust. Plants P3 and P4 also were infected with aphids, each plant with >35 insects. Both Marchionese of Londonderry plants (P5 and P6) were rated 5 for powdery mildew and rust after Paulus and Nelson (*supra*).

Two treatment formulae were used for this trial. Each plant (P1 through P6) received a ≈100ml treatment spray of the formulation as indicated in Table 1 below. Control plants were sprayed with water alone. The change in the rating from pre-treatment to post-treatment was calculated as the mean percentage of disease control (MPDC) as described herein.

Table 1

| Plant - Treatment/Dose Assignment | |
|---|---|
| Treatment/Dose | Plant |
| T1 - Low | P1, P4, P6 |
| T2 - High | P2, P3, P5 |

As shown in Table 2 below, both formulas reduced levels of infection. Both powdery mildew and rust levels of infection were reduced a minimum of one rating category after treatment as compared to plants sprayed with water alone. Aphids were eliminated from P3 and P4, indicating that the formulas have insecticidal properties. Coniferyl aldehyde, as does cinnamic aldehyde, has antibiotic properties and may eliminate symbiotic bacteria present in the host insect without which the insect cannot live.

Table 2

| Treatment of Rose Plants Pathogens with Coniferyl Aldehyde | | | | | | |
|---|---|---|---|---|---|---|
| | Plant | | | | | |
| | Treatment/Dose Assignment | | | | | |
| | Low (T1) | | | High (T2) | | |
| PEST | | | | | | |
| | P1 | P4 | P6 | P2 | P3 | P |
| | | | | | | 5 |
| Powdery Mildew | | | | | | |
| Pre (rating) | 3 | 3 | 4 | 5 | 5 | 5 |
| Post (rating) | 2 | 1 | 1 | 2 | 1 | 1 |
| Change | 1 | 2 | 3 | 3 | 4 | 4 |
| Rust Pre | 3 | 3 | 4 | 5 | 5 | 5 |
| (rating) Post | 2 | 1 | 3 | 2 | 1 | 1 |
| Change | 1 | 2 | 1 | 3 | 4 | 4 |
| Aphids Pre (#) | - | 35 | | - | ≥35 | - |
| Post (#) | - | 0 | | - | 0 | - |
| Change | - | 35 | | - | ≥35 | - |

Example 3

Treatment of Powdery Mildew on Rose

A three treatment experiment with cinnamic aldehyde formula, coniferyl aldehyde formula and combined cinnamic and coniferyl aldehyde formula was carried out on field grown roses known to be susceptible to powdery mildew. The plants were blocked by variety before fungicide treatments and were randomized as to the plants. Two varieties were used in each of the three experiments described below. In experiment 1, Reichsprasident von Hindenburg (Bourbon) and Oskar Cordel (Hybrid Perpetual) were used; in experiment 2, Rosa Gallica Officinalis (Apothecary Rose) and Deuil de Paul Fontaine (Hybrid Moss) were used. In experiment 3 Comte de Chambord (Portland) and Madame Pierre Oger (Bourbon) were used. In Experiment 1, the effect of cinnamic aldehyde alone and in combination with Tween 80 and/or $NaHCO_3$ component was evaluated in experiment 2 the effect of coniferyl aldehyde atone and in combination with Tween 80 and/or $NaHCO_3$ was evaluated and in experiment 3 a combination of cinnamic aldehyde and coniferyl aldehyde with Tween 80 and/or $NaHCo_3$ was evaluated. Nine treatments were tested in experiment 1, six in experiment 2 and six in experiment 3. *See* Table 3 for the treatment protocol; formula 1 was used for these experiments. Each plant received a single foliar spray of 100ml following evaluation of powdery mildew infestation using the Paulus/Nelson rating scale *(supra.)*.

Plants were evaluated using the Paulus/Nelson scale just prior to and four days after treatment. Mean percentage of disease control data indicate that all three combination formulas (i.e. G, M, and Q) provided in excess of 70% disease control based on these experiments (Table 4). Treatment Q (both CNMA and COFA) was significantly better than all other treatments, including benomyl. Moreover, cinnamic aldehyde, coniferyl aldehyde, Tween 80 and $NaHCO_3$ are used in the food industry and there is likely to be little toxicological risk to the consumer or handler from any horticultural or food crop sprayed in this way. Similarly, as these chemicals leave no toxic residue, there is little chance of any detrimental effect on the wider environment, and their use is likely to be compatible with current biological control methods such as beneficial insects and/or microorganisms.

Table 3

Treatment Protocol

| Group | Treatment | Ingredient(s) | Amount of treatment ingredient(s)[1] a | b |
|---|---|---|---|---|
| 1 | A | Cinnamic aldehyde (CNMA) | 5g | 20g |
| 1 | B | Tween 80 (T80) | 10g | 20g |
| 1 | C | $NaHCO_3$ | 80g | 60g |
| 1 | D | CNMA+T80 | 5g, 10g | 20g, 60g |
| 1 | E | CNMA+$NaHCO_3$ | 5g, 80g | 20g, 60g |
| 1 | F | $NaHCO_3$+T80 | 80g, 10g | 60g, 20g |
| 1 | G | Formula 1 (CNMA) | A=5, B=10g, C=80g | A=20, B=20, C=60g |
| 1,2,3 | H | +Control[2] | per manufacturer instructions | R,S,T[2] |
| 1,2,3 | I | -Control | $H_2O$ | $H_2O$ |
| 2 | J | Coniferyl aldehyde (COFA) | 5g | 20g |
| 2 | K | COFA+T80 | 5g, 10g | 20g, 20g |
| 2 | L | COFA+$NaHCO_3$ | 5g, 80g | 20g, 60g |
| 2 | M | Formula 2 (COFA) | J=5g, B=10g, C=80g | J=20g, B=20g, C=60g |
| 3 | N | CNMA+COFA | 2.5g, 2.5g | 10g, 10g |
| 3 | O | CNMA+COFA+T80 | 2.5g, 2.5g, 10g | 10g, 10g, 10g |
| 3 | P | CNMA+COFA+$NaHCO_3$ | 2.5g, 2.5g, 80g | 10g, 10g, 60g |

| Group | Treatment | Ingredient(s) | Amount of treatment ingredient(s)[1] | |
|---|---|---|---|---|
| | | | a | b |
| 3 | Q | Formula 3 (CNMA+COFA) | A=2.5g, J=2.5g, B=10g, C=80g | A=10g, J=10g, B=20g, C=60g |

Table 4

Effect of Cinnamic Aldehyde and Coniferyl Aldehyde Formulations on Rose Powdery Mildew

| Additive Formulation | Aldehyde | | | |
|---|---|---|---|---|
| | None | Cinnamic Aldehyde (5g) | Coniferyl Aldehyde (5g) | Cinnamic Aldehyde (2.5g) + Coniferyl Aldehyde (2.5g) |
| | Mean % Disease Control | | | |
| None | 0% | 50% | 56% | 69% |
| T80 (10g) | 0% | 44% | 44% | 69% |
| $NaHCO_3$ | 44% | 56% | 44% | 88% |
| T80 + $NaHCO_3$ | 19% | 94% | 81% | 100% |
| Benomyl | 79% | NT | NT | NT |

* NT = not tested

Treatment of Grape Phylloxera with Cinnamic Aldehyde alone and/or with Tween 80 and/or $NaHCO_3$

Feeding Site Location Test Mortality resulting from physiological process disruption was determined by the adult and nymphal mortality experiment and by the egg hatch experiment. After hatching, new insects must secure an appropriate feeding site. This activity must be successful if the life cycle of the insect is to continue. Research indicates that approximately 80% of phylloxera mortality occurs during this activity. Low dose concentrations of formulae may

be protective of grape stock roots by disrupting the "search and identify feeding site" behavior of the insect.

Adult and Nymphal Mortality Experiment Approximately twenty four eggs of phylloxera were allowed to develop for up to 30 days on standard excised grape roots. At around 30 days, some of the insects were nymphs while others were adults. New eggs were removed during the process. Insect infected roots were submerged into a test formula for 6 seconds then set aside to dry in the air. The percentage of live insects, as defined by growth, oviposition or limb movement, was determined after 5 days. An insect was considered dead if it abandoned its feed site.

In an initial test, doses of 20,000 ppm cinnamic aldehyde in water (*i.e.*, 2% cinnamic aldehyde) with various additives were evaluated. Cinnamic aldehyde without any additives produced 83.3% mortality. With 1% Tween 80 added, 91.7% mortality was seen. With 6% $NaHCO_3$ added to a solution of 2% cinnamic aldehyde, 91.7% mortality was seen. With 1 % Tween 80 and 6% $NaHCO_3$ added to a solution of 2% cinnamic aldehyde, 100% mortality was seen. Water with no additives produced no mortality, while a positive control solution of 250 ppm malathion in water gave 100% mortality.

Egg Hatch Experiment Mixed age groups of 60 grape phylloxera eggs were established on filter paper (Whatman #1.5.5 cm circles) in 50x9 mm sealed plastic petri dishes treated with 100 μl of solution. A selected concentration of a test formulation of 400 μl was added to the filter disk and the dish closed with the petri dish cover and placed in a plastic container box. After 6 hours, the box was placed in an environmental chamber at 24°C in the dark. The eggs were placed in groups of 10. After one week, the percentage of hatch is determined. In an initial test, doses from 0.1 to 25,000 ppm cinnamic aldehyde in 6% $NaHCO_3$ and 2% Tween 80 were evaluated with a single group of eggs at each dosage. Three replicates of the experiment were performed. The effects of the formulation were evaluated after 7 days and scored as the number of nymphs that died in the shell (DIS), or eggs that did not hatch completely (IH) (*i.e.*, all died). LD50 and LD95 were determined by probit analysis. At 5000 ppm cinnamic aldehyde, all nymphs died in the shell. At 100 ppm, 88% died in the shell and the remaining 12% did not hatch completely. At 10 ppm, none died in the shell, but 100% of the eggs did not hatch completely. The addition of 0.86 ml of a $10^0$ brix saponin solution in water to the formulation at 100 ppm increased the number of nymphs which died in the shell to 93%. Coniferyl aldehyde over the same dosage range (in 6% $NaHCO_3$, 2% Tween 80) was less potent. Although 10% of nymphs died in the shell at 5000 ppm, at 1000 ppm, 12% died in the shell and 85% hatched incompletely. All phylloxera eggs treated with $H_2O$ alone hatched; 100% of those treated with Carbofuran (10 ppm) or malathion (250 ppm) died in the shell.

Grape phylloxera eggs were treated with cinnamaldehyde (CNMA) in a 6% $NaHCO_3$, 2% Tween-80 formulation in a laboratory bioassay. Mortality of the eggs was indicated by failure of the egg shell to split. The 7 day $EC_{50}$ was 115 ppm CNMA. The $LC_{50}$ for root dip treatments of nymphs and adults after 7 days was between 3,000 ppm and 10,000 ppm. Leaves of one-year-old greenhouse *Vitis vinifera* cultivar Merlot vines heavily infested with grape phylloxera were sprayed to run-off with a CNMA treatment using the same formulation. After two weeks, insects had disappeared from the roots of all the treated vines but not from the water controls (Table 5). Roots taken from plants 1.2 and 5 weeks after CNMA or other treatments were placed in Petri dish bioassays and infested with untreated phylloxera eggs. These eggs established new phylloxera populations on the roots from the water-treated control plants, but not from the CNMA-treated plants (Table 5). This result indicated long-term efficacy of the treatment. One year old Merlot vines (Table 6) and 2-year-old Chardonnay plants (Table 7) grown in field, planter boxes in Davis, CA, were sprayed with CNMA (in 1% Tween 80), water control or formula blank. After two weeks, the 10,000 ppm treatments had a 95% reduced population from the population on the control vines; 1,000 ppm treatments resulted in a 92% reduction (Table 8). These results indicate that root forms of grape phylloxera are subject to control by this agent applied to the leaves.

CNMA may be directly toxic to phylloxera, as indicated by the laboratory data, and/or CNMA and/or its metabolites may initiate a hormonal or waxed-healing change in the plant's physiology that is detrimental to phylloxera survival.

## Example 5

### Chemical Treatments of Grapevine Leaves for Control of Root Forms of Grape Phylloxera

The root form of grape phylloxera (*Daktulosphaira vitifoliae* Fitch) is arguably the most devastating grape insect world wide. A laboratory colony of biotype A and B phylloxera was maintained on excised *V. vinifera* cultivar Cabernet Sauvignon root pieces as described previously (DeBenedictis and Granett (1992). Eggs from the colony were used for toxicity studies, for infesting greenhouse and field box plants, and for evaluating host suitability of chemically treated plants. Laboratory bioassays were conducted similarly to tests described by Granett and Timper (1987).

In this experiment, cinnamic aldehyde formulated with 1% Tween 80 was tested for its ability to control phylloxera. In greenhouse and planter box studies, this basic formulation was augmented with various concentrations of sodium bicarbonate, technical grade. Field treatments were with 1% cinnamic aldehyde plus 1% Tween 80 and with 1% Tween 80 alone. Also tested were analogs and other materials, including cinnamyl alcohol, cinnamyl acid methyl ester, α-hexyl cinnamylaldlehyde, phenylalanine, and 4-acetamidophenol (98%, Aldrich Chemical Co., Inc. Milwaukee,

WI).

Eggs of various age classes were placed on Whatman #1 filter paper moistened with enough treatment solution to form a meniscus about halfway up the sides of the eggs. The pieces of filter paper with eggs were maintained in sealed plastic Petri dishes at 2°C until they were 7 days old and then hatched individuals were counted. Hatch was considered successful if the nymph had completely extracted itself from the chorion. Insects were considered unhatched even if they were partially emerged from the chorion. The results are shown in the Tables below.

Table

| Egg tests with CNMA. 24 hr treatments by placing eggs each day on CNMA moistened filter paper; eggs held at 24°C. Mortality was recorded after 24 hr. of contacting egges with the filter paper. | | | | | |
|---|---|---|---|---|---|
| Approximate Percent Mortality | | | | | |
| Days of Treatment | | | | | |
| CNMA Conc. (ppm) | | | | | |
| | 1 | 2 | 3 | 4 | 5 |
| 100 | 8 | 0 | 0 | 6 | 25 |
| 180 | 100 | 0 | 0 | 47 | 72 |
| 320 | 100 | 37 | 28 | 85 | 100 |

Table

| Egg tests with CNMA analogs. 24 hr treatments by placing eggs on CNMA or analog-moistened filter paper | |
|---|---|
| Compound | approximate egg hatchLD 50 |
| Cinnamic aldehyde | 320 ppm |
| Cinnamyl alcohol | >320, <560 ppm |
| Cinnamyl acid methyl ester | >320, <560 ppm |
| α Hexyl cinnamylaldehyde | no hatch failure at 560 ppm; rapid nymphal mortality, 100% at 100 ppm |
| 4-acetamidophenol | no mortality at 560 ppm |

Table 5

Greenhouse Merlot Plants, 1 year old, infested with phylloxera - biotype A

| Date and Activity[1] | 10 K CNMA | 3 K CNMA | 2.5 K CNMA | 1 K CNMA | 0.3 K CNMA | Water | 200 ppm Malathion | Formulation Blank | Clipped |
|---|---|---|---|---|---|---|---|---|---|
| 0 days, Treatments | 4 | 4 | | 4 | 4 | 4 | | | |
| 7 day evaluation | 0[2](3)[3] | 0(3) | | 0.7(3) | 3.3(3) | | | | |
| Set up clipped plant at day 7 | | | | | | | | | 3 |
| 14 day evaluation | 0(3) | 0(3) | | 0(3) | 0(3) | 3.0(3) | | | 3 |
| 5 week post treat | 0(3) | 0(3) | | 0(3) | 0.7(3) | 4.0(3) | | | |
| Set up Petri Dish | | | 4 | | | 4 | 4 | 4 | |
| 1 day post treat. | | | 4.0(3) | | | 4.0(3) | 4.0(3) | 4.0(3) | |
| 2 days post treat. | | | 0.3(3) | | | 4.0(3) | 2.3(3) | 0(3) | |
| 7 days post treat. | | | 0.3(3) | | | 3.3(3) | 0(2) | 1.7(3) | |

[1]Treated with concentrations of CNMA in 6% $NaHCO_3$ and 2% Tween -80 to run-off. [2]Average of 0-4 disease rating. [3](number of plants).

Table 6

Merlot Plants, 1 year old, infested with phylloxera Biotype A

Number of Plants

| Date and Activity | 20 K* | 10 K | 10 K painted | 1 K | Water | Form. blank |
|---|---|---|---|---|---|---|
| Treat (5:30 p.m.) | 2 plants | 4 plants | 2 plants | 3 plants | 3 plants | 2 plants |
| 7 days post treat. | 1 plant | 2* plants | 1 plant | 1 plant | 1 plant | 1 plant |
| 14 days post treat | 1 plant | 2 plants | 1 plant | 2 plants | 2 plants | 1 plant |

* one 10 K plant and formulation blank covered with wood in a box.

** 20,000 ppm of CNMA in 6% $NaHCO_3$ and 2% Tween -80.

Table 7

Field Box Tests Chardonnay, 2 years old, infested with biotyope A

Number of Plants

| Date and Activity | 10 K CNMA* | 1 K CNMA | Water | Form. Blank |
|---|---|---|---|---|
| 0 days treatment | 2 plants (box 5) | 2 plants (box 2) | 2 plants (box 1) | 2 plants (box 6) |
| 7 days evaluation | 1 plant | 1 plant | 1 plant | 1 plant |
| 14 days evaluation | 1 plant | 1 plant | 1 plant | 1 plant |

* 10,000 ppm CNMA in 6% $NaHCO_3$ and 2% Tween -80.

Table 8

| Phylloxera population in plant roots | | |
|---|---|---|
| Number of phylloxera per root piece | | |
| Two-weeks results | Merlot | Chardon nay (2 treatmen ts) |
| 20 K* | 16 | |
| 10 K | 96, 52, 33 | 102 |
| 1 K | 133, 72, 95 | 637 |
| Water Control | 1143, 1138 | 1048 |
| Formula Blank | 1279 | 2521 |

* 20,000 ppm of CNMA in 6% $NaHCO_3$ and 2% Tween -80.

For nymph and adult tests, eggs were placed on excised root pieces similar to those used for rearing purposes and these were sealed in Petri dishes and held at 24°C for 18-25 days to allow insects to hatch, initiate feeding sites and develop. At about this time about half the population was in the adult stage. Prior to testing, the population on each root was determined as well as the developmental stage of each insect. Eggs were removed. These root pieces were then dipped for 5 sec into test solutions and allowed to air dry before they were returned to Petri dishes. sealed and placed back in the temperature chamber at 24°C. Mortality was determined after 5 days. Insects were considered dead if they appeared black or desiccated and if they had not developed since the pre-treatment reading and had laid no or few new eggs.

Nymph/adult test. An average 2% decrease in phylloxera was observed for the water control, suggesting that the formulation blank, Tween 80, is somewhat toxic to the nymphal and adult phylloxera.

For greenhouse tests, one year old potted *V. vinifera* cultivar merlot plants were used that had been obtained from Foundation Plant Material Service, University of California, Davis. The plants were potted in 10 cm diameter pots and were infested with phylloxera 5 weeks prior to treatment. At the time of spraying with a treatment solution, plant pots were placed in a plastic bag so that chemical runoff from the leaf sprays would not drip into the soil. The plants were sprayed to runoff with a household 1 liter-capacity plastic spray bottle. Plants were allowed to air dry outside and then replaced in the greenhouse. At intervals thereafter, the roots were separated from the potting mix and total phylloxera population estimated.

| Greenhouse Merlot plants, 1 year old, Experiment 1, leaf treatments (n=3) Average disease rating (disease rating 0-4) | | | | | |
|---|---|---|---|---|---|
| Week | 0 ppm | 300 ppm | 1000 ppm | 3000 ppm | 10,000 ppm |
| 1 | 3.3 | 0.7 | 0 | 0 | 0 |
| 2 | 3.0 | 0 | 0 | 0 | 0 |
| 5 | 4.0 | 0.7 | 0 | 0 | 0 |

Roots from plants at 5 weeks post-treatment were excised and 6 root sections, about 3 mm diameter by 4 cm length were cleaned of infestations and used in a bioassay. For this bioassay, root sections were inoculated with 20 eggs and the roots maintained as the original colonies were maintained *(see* description above). After 25 days, the number of individual phylloxera of each age class were counted.

| Reinnoculation tests with roots taken 5 weeks after treatment (25 day bioassay), Total number of phylloxera on 6 roots per dosage. | | | | | | |
|---|---|---|---|---|---|---|
| Treatment | Eggs | 1st instar | 2nd instar | 3rd instar | 4th instar | Adults |
| Water | 52 | 7 | 7 | 7 | 13 | 16 |
| 300 ppm | 16 | 0 | 6 | 5 | 0 | 4 |
| 1000 ppm | 2 | 0 | 1 | 4 | 2 | 1 |
| 3000 ppm | 0 | 0 | 2 | 1 | 1 | 0 |
| 10,000ppm | 0 | 0 | 2 | 0 | 0 | 0 |

Planter boxes are wooden boxes with a top surface area of 1.5 m x 1 m and are 1.5 m tall. They were filled with a clay-loam soil and each was planted to 4 one-year old Merlot plants in early spring, 1995. They were infested with biotype A phylloxera in May, 1995 by placing about 150 eggs adjacent to an attached root under the soil surface. The plants were irrigated weekly with a fine mist. Treatments were made at various times in July and August 1995 using the household 1 liter-capacity plastic spray bottles, spraying to runoff. At one or two weeks after treatment, plants root systems were removed from the soil and total phylloxera populations were estimated.

| Greenhouse Merlot plants, 1 year old, Experiment 2, leaf treatment Average disease rating (n) | | | | |
|---|---|---|---|---|
| Day post treatment | Water | Form. Blank | 2500 ppm CNMA | 200 ppm Malathion |
| 1 | 4.0 (3) | 4 (3) | 4 (3) | 4 (3) |
| 2 | 4.0 (3) | 0 (3) | 0.3(3) | 2.3 (3) |
| 7 | 3.3 (3) | 1.7 (3) | 0.3 (3) | 0 (2) |

| Boxed plants, 1 year old Merlot plants, CNMA treatment of foliage to runoff. Plants with poor, disconnected roots excluded | | | | |
|---|---|---|---|---|
| | Exp. 1 | | Exp. 2 | |
| | Mean ±95% CI | (n) | Mean ±95% CI | (n) |
| Control | 1187±91 | (3) | 767±135 | (6) |
| 1000 ppm | 100±31 | (3) | 58±17 | (5) |
| 10,000 ppm | 60±37 | (3) | 43±56 | (2) |

Direct toxicity The above results demonstrate that CNMA is directly toxic to grape phylloxera eggs, nymphs and adults when applied topically. The level of toxicity to the feeding stages is much lower than the toxicity to the egg stage. Eggs were more sensitive when young or when near hatch. These toxicity differences could reflect absoprtion characteristics, detoxifying enzymes or site of activity differences among the stages of development.

Systemic activity When foliage of whole plants was treated either in the greenhouse or in the field, activity was translocated to the roots within two weeks. Vacant feeding sites were seen on the roots and the excised roots maintained resistance to phylloxera reinfestation for at least 5 weeks after treatment, suggesting that the aromatic aldehyde and/or a metabolite is translocated to the roots where it directly causes phylloxera to die and/or vacate feeding sites. Alternatively, the aromatic aldehyde induces the plant to change its root chemistry in a way that makes the roots unacceptable to phylloxera feeding. Either mechanism is an exciting new approach to control of grape phylloxera and other pest species. Conventional systemic insecticides generally are upwardly mobile in plants, not downwardly mobile; therefore this downward mobility is an important addition to the insecticidal arsenal, and adds a new approach to treatment of plant pests.

Example 6

Protocol for Aphid and White Fly

Activity of cinnamic aldehyde and/or coniferyl aldehyde against black bean aphid, *Tetranychus urticae*, and silverleaf white fly, *Bemisia argentifolii* was determined as follows:

Aphids

Petri Dish Bioassay Petri dishes (60 mm) were treated with cinnamic aldehyde at 10-25,000 ppm in 2% Tween 80 and 6% $NaHCO_3$ dissolved in water, and the dishes allowed to air dry. Twenty adult aphids (*Tetranychus urticae*) were put in each dish, (replicate 10 times). The mortality after three hours in contact with a treated plate was compared to that of aphids in petri dishes treated only with diluent. Malathion (250 ppm) was used as a positive control. At concentrations of 2500 ppm cinnamic aldehyde and above, 100% of the aphids were killed. At 100 ppm and 10 ppm, 50% and 25% mortality was observed. Twenty-five percent mortality was observed with the concentration of 2% Tween 80 and 6% $NaHCO_3$ (no aldehyde added), and 100% of aphids were killed with malathion (250 ppm).

Plant Foliar Bioassay Plants are grown in 7.5 mm pot in potting soil in greenhouse. Cotton plants are used for white fly and sugar beets are used for aphids. When plants reach 3 leaf stage, they are infested with 60 of the

specified anthropod (6 replications). The insect is allowed to settle and feed. The plant is sprayed to runoff (about 5 ml) with a formulation containing 100 to 2000pm, or 0.1 to 2 g/l concentration of a test formulation. The plant is covered with tall plastic cage (5 mm tall x 10 mm diameter). The mortality of the insects after three days on the plants sprayed with a test formulation is determined and compared with that of insects on plants sprayed only with water.

Silver Leaf White Fly

Petri Dish Bioassay Petri dishes (60 mm) were treated with cinnamic aldehyde at 10-25,000 ppm in 2% Tween 80 and 6% $NaHCO_3$ dissolved in water, and allowed to air dry. Twenty adult silver leaf white fly were put in each dish, (replicate 10 times). The mortality after three hours in contact with a treated plate, was compared to that of silver leaf white fly in petri dishes treated only with diluent. Malathion at 250 ppm was used as a positive control. At concentrations of 2500 ppm and above, 100% of the silver leaf white fly were killed. At 100 ppm and 10 ppm, 50% and 25% mortality was observed, respectively. Twenty-five percent mortality was observed with the concentration of 2% Tween 80 and 6% $NaHCO_3$ (no aldehyde added), and 100% of silver leaf white fly were killed with malathion (250 ppm). *See* Table 9.

Table 9

| Effect of Cinnamic Aldehyde and Coniferyl Aldehyde Formulations on Silver Leaf White Fly Mortality (Percent) | | |
|---|---|---|
| Additive Formulation | None | Cinnamic Aldehyde (20g) |
| None | 0 | 68.6 |
| T80(10g) | 14.5 | 72.1 |
| $NaHCO_3$ | 22.9 | 87.3 |
| T80(2%)+ $NaHCO_3$ (6%) | 25.0 | 100 |
| Malathion (250 pgm) | 100 | NT* |
| $H_2O$ (Neg. Control) | 26.9 | NT* |

• NT: not tested

Example 7

Treatment of Nematode Infestation

Various kinds of nematodes infest plant tissue, including the stem and bulb nematode *(Ditylenchus dipsaci)* and rootknot nematode *(Meloidogyne spp.).* The treatment of stem nematode *(Ditylenchus dipsaci)* with various formulations containing cinnamic aldehyde was tested as follows.

Stem nematodes Stem nematodes were extracted from garlic cloves by chopping the tissue into a mesh-bottomed beaker and suspending the mesh-bottomed beaker in a beaker of water. Nematodes migrate from the host tissue and sink down through the mesh into the bottom beaker. The supernatant water is removed and the nematodes remaining in the beaker are transferred to a watchglass and used in the treatment protocol as follows. Clear plastic trays are divided into open-topped cells measuring 20 mm x 20 mm x 20 mm. One half ml of tapwater at room temperature (19°C) is pipetted into each cell. Ten nematodes are placed in each cell using an eyelash glued to a dissecting needle to handle each animal. One-half ml of one test solution is then added to each cell. Water is added to the control wells. Survival of nematodes in the cell is monitored by observation using a binocular microscope. The number of animals surviving 1, 5, 10, 20, 30 and 60 minutes after addition of the solutions is recorded. Mortality is assumed if individual nematodes are immobile and fail to respond to manipulation. The test is repeated three times.

Root nematodes

Petri dish assay In a double blind study, concentrations of the subject aldehyde compounds were tested for activity against root-knot nematode, *Meloidogyne javanica.* Nematodes were put in direct contact with the chemical and at 24 hour intervals, mortality was assessed both visually and by probing. *Meloidogyne javanica* were produced using hydroponics. The nematodes were harvested and used within 24 hours.

Approximately 100 nematodes in 0.07 mls of water were pipetted into a syracuse dish (Fisher) and 1 ml of test formulation was immediately pipetted into each dish. The dishes were then placed into plastic bags to retain moisture and prevent evaporation. Four syracuse dishes were used for each solution test formulation. Every 24 hours for 7 days, the solutions were examined and the first 10 nematodes encountered were assessed as either living or dead, based on morphological integrity of the nematode and touch. Moving nematodes were counted as living.

At concentrations greater than 100 ppm cinnamic aldehyde in vehicle (2% Tween 80, 6% $NaHCO_3$), 100%

nematodes were dead at 24 hours. At 10 ppm, 0%, 15%, 17.5% 22.5%, 27.5%, 52.5% and 52.5% were dead at 24, 48, 72, 96, 108, 132, and 156 hours, respectively. There was no effect on mortality at 1 ppm and 0.1 ppm cinnamic aldehyde in vehicle. Addition of a 1:60 dilution 10 brix concentrate of *Yucca shidigera* saponin resulted in 100% mortality at 24 hours with the lowest concentration of cinnamic aldehyde in vehicle tested, 0.1 ppm. However, saponin alone had the same effect. Ethanol (95%) killed all nematodes at 24 hours. Minimal effect of the vehicle on mortality was observed: 2.5% at 72 hours and 5% at 108 hours.

Plant Foliar Bioassay. The subject formulations were tested for ability to reduce grape vine infestation by root knot, ring, stubby root, and roll lesion nematodes. Grape vines (Harmony rootstock) in a vinyard were treated with 1000 ppm or 3000 ppm cinnamaldehyde in 2% Tween 80, the commercial anti-nematode agent Nemacur, or a formulation blank. The extent of nematode infestation was determined at the time of treatment and at 30 and 60 days post-treatment. The results are shown in Table 10.

Table 10

Effect of Cinnamaldehyde on Nematode Infestation of Grape Vines

| | Nematodes per 250 cc soil[1] | | | | | | | | | Nematodes per gram root[2] | | | | | |
|---|---|---|---|---|---|---|---|---|---|---|---|---|---|---|---|
| | Root Knot Nematode | | | Ring Nematode | | | Stubby Root | | | Root Knot | | | Roll Lesion | | |
| Treatment | Days Post-Treatment | | | Days Post-Treatment | | | Days Post-Treatment | | | Dates Post-Treatment | | | Days Post-Treatment | | |
| | 0 | 30 | 60 | 0 | 30 | 60 | 0 | 30 | 60 | 0 | 30 | 60 | 0 | 30 | 60 |
| Untreated | 2110.8 | 1360 | 276.8 | 117.5 | 151.5<br>179.6 | | 39.8 | 10.8 | 44.5 | 393.2 | 130.8 | 25 | 0 | 0.02 | 0 |
| 1000 ppm cinnamic aldehyde* | 498.8 | 872 | 204.5 | 47.2 | 171.2 | 1168 | 13.8 | 45 | 36.2 | 301.3 | 55.2<br>11.8 | | 2.9 | 2.7 | 0 |
| 3000 ppmcinnamic aldehyde* | 379.8 | 700 | 269.8 | 540.2 | 322.8 | 1127 | 0.8 | 8.8 | 43.5 | 24.4 | 125.6 | | 1.0 | 0.56 | |
| Nemacur | 1008 | 568.8 | | 139.8 | 181.5 | | 3 | 17.8 | | 36.5 | 72.4 | 36 | 0.4 | 0.08 | |
| Formulation Blank | 332.5 | 935 | | 58.2 | 19 | | 7.2 | 17.2 | | 36.5 | 93.2 | | 22.8 | 1.1 | |

* in 2% Tween 80

[1] Nematodes extracted by sieve-sugar centrifugation.

[2] Washed roots misted for 5 days to extract juveniles.

Example 8

Treatment of Strawberry Red Core (*Phytophthora Fragariae*)

Strawberry red core disease is caused by the fungus *Phytophthora fragariae* Hickman which is spread by means of infected planting material or soil infested with long-lived oospores of infected debris. Various formulations containing cinnamic aldehyde and/or coniferyl aldehyde are tested as follows. Macerated strawberry roots infected with *Phytophthora fragariae* are thoroughly mixed with infested compost and allowed to decompose for 4 to 6 weeks to produce a well rotted inoculum for treatment, This is divided into 1 kg lots and mixed with 1500 ml of a test formulation at different concentrations. After 10 minutes of treatment, the compost is rinsed under running tap water on a 25 mm sieve for a minimum of 5 minutes to remove all traces of the test formulation. The compost is then put into 9-cm diameter plastic pots and planted with 4 strawberry plants per pot. Five pots are used for each treatment. Plants are grown in a controlled environment room at 15°C and 18 h daylength; the compost is kept damp to encourage infection. Pots are placed on grids to avoid cross infection among treatments.

After 9 weeks the strawberry plant roots are washed free of compost and examined for signs of infection by cutting roots longitudinally and looking for red steles, and rotted or brown roots. All infections are confirmed by microscope examination of root pieces for the presence of oospores of *Phytophthora fragariae*.

Example 9

Stability of Cinnamic Aldehyde

Protocol to determine the stability of cinnamic aldehyde with and without an anti-oxidant over time

Cinnamic aldehyde at 2% by weight is added to a formula containing 2% Tween 80 and 6% sodium bicarbonate with and without the addition of vitamin E (tocopherol at 1% of the CNMA concentration). The solutions are maintained at 50°C for two weeks. The solutions are analyzed for cinnamic aldehyde concentration on regular intervals during the two week period by HPLC/UV and recorded (high pressure liquid chromatography ultra violet detection).

Example 10

Pitch Canker Disease

Pitch canker disease, caused by the fungus *Fusarium subglutinans* f. sp. *pini* is characterized by a resinous exudation on the surface of shoots, branches, exposed roots and boles of infested trees. The host and geographic range of the pitch canker pathogen has greatly increased since it was first discovered in California in 1986. The pathogen has recently been discovered in Mexico and Japan.

A double-blind bioassay was undertaken using cinnamic aldehyde in various concentrations and formulations. The bioassay was based on inhibition of radial growth of *Fusarium subglutinans* f. sp. *pini.* Eight ml of formulation (concentration unknown) was pipetted into 200 ml of molten 2% potato dextrose agar (PDA) and the mixture was dispersed into five plastic petri dishes (25 ml dish). Each of four plates was inoculated at the center with an agar plug transferred from a growing PDA culture of *Fusarium subglutinans* f. sp. *pini* (isolate SL-1, UCB), while the fifth plate was left noninoculated as a control. These steps were repeated for each formulation of the cinnamic aldehyde. As a positive control, four PDA plates amended with 5 ppm benomyl were inoculated, as described; the negative control was four unamended PDA plates that were inoculated with *F. subglutinans.* All inoculated and noninoculated plates were incubated at 18°C for five days, after which colony diameters were measured.

Table 11 shows the colony diameter raw data averages for the bioassay, and Table 12 compares the effect on colony diameter of CNMA at various concentrations, with and without saponin (*Yucca schidegira* extract 10° BRIX at 0.86 ml).

Table 11

| Radial Growth of Fusarium subglutinans f. sp. pini | |
|---|---|
| (Data Averages)* | |
| Treatment | Colony diameter (cm) |
| PDA (unamended) | 4.938 |
| Formula Blank | 4.380 |

Table 11 (continued)

| Radial Growth of Fusarium subglutinans f. sp. pini | |
|---|---|
| (Data Averages)* | |
| Treatment | Colony diameter (cm) |
| 10 ppm CNMA | 4.363 |
| 10 ppm CNMA + Saponin | 3.600 |
| 100 ppm CNMA | 4.238 |
| 100 ppm CNMA + Saponin | 4.300 |
| 2,500 ppm CNMA | 3.513 |
| 2,500 ppm CNMA + Saponin | 3.600 |
| 5,000 CNMA | 2.908 |
| 5,000 ppm CNMA + Saponin | 2.913 |
| 12,500 ppm CNMA | 0.000 |
| 12,5000 CNMA + Saponin | 0.000 |
| 25,000 CNMA | 0.000 |
| 25,000 CNMA + Saponin | 0.000 |
| $H_2O$ | 3.738 |
| Saponin | 4.138 |
| 5 ppm Benomyl (Positive Control) | 0.000 |
| Glutaraldehyde 2% | 3.663 |

Table 12

| Radial Growth of F. subglutinans f. sp. Pini | | |
|---|---|---|
| | Treatments | |
| | PGXL CNMA | PGXL CNMA + Saponin (.86 ml) |
| ppm | colony diameter (cm) | colony diameter (cm) |
| 10 | 4.36 | 3.60 |
| 100 | 4.24 | 4.30 |
| 2,500 | 3.51 | 3.60 |
| 5.000 | 2.91 | 2.91 |
| 12,500 | 0 | 0 |
| 25,000 | 0 | 0 |
| Controls | colony diameter (cm) | |
| 2% Glutaraldehyde | 3.66 | |
| $H_2O$ | 3.74 | |
| 5 ppm Benomyl | 0 | |
| PDA (unamended) | 4.04 | |

Example 11

Treatment of Corn Root Worm with Cinnamic Aldehyde and with Tween 80 and/or $NaHCO_3$

Plant Foliar Bioassay Plants are grown in 7.5 mm pots in potting soil in a greenhouse. Corn plants are used for corn root worm. When plants reach 3 leaf stage, they are infested with 60 of the specified arthropod (6 replications). The corn root worm is allowed to settle and feed. The plant is sprayed to runoff (about 5 ml) with a formulation containing 100 to 2000 ppm, or 0.1 to 2 g/l concentration of a test formulation. The plant is draped with plastic covering to prevent the formulation from touching the soil. The mortality of the worms after three, five and seven days on the plants sprayed with a test formulation is determined and compared with that of worms on plants sprayed only with water and/or a formula blank.

Example 12

Treatment of Russian Wheat Aphid with Cinnamic Aldehyde and with Tween 80 and/or $NaHCO_3$

Plant Foliar Bioassay Plants are grown in 7.5 mm pots in potting soil in a greenhouse. Wheat plants (Kansas variety) are used for russian wheat aphid. When plants reach 3 leaf stage, they are infested with 60 of the specified arthropod (6 replications). The insect is allowed to settle and feed. The plant is sprayed to runoff (about 5 ml) with a formulation containing 100 to 10,000 ppm, or 0.1 to 10 g/l concentration of a test formulation. The plant is draped with plastic covering to prevent the formulation from touching the soil. The mortality of the insects after 36 hours, five days and seven days on the plants sprayed with a test formulation is determined and compared with that of insects on plants sprayed only with water and/or a formula blank.

Example 13

Treatment of Thysanoptera with Cinnamic Aldehyde and with Tween 80 and/or $NaHCO_3$

Plants are grown in 7.5 mm pots in potting soil in a greenhouse. Rose plants of various varieties are used for aphids. When plants reach 3 leaf stage, they are infested with 60 of the specified arthropod (6 replications). The insect is allowed to settle and feed. The plant is sprayed to runoff (about 5 ml) with a formulation containing 100 to 10,000 ppm, or 0.1 to 10 g/l concentration of a test formulation. The plant is draped with plastic covering to prevent the formulation from touching the soil. The mortality of the insects after 36 hours, five days and seven days on the plants sprayed with a test formulation is determined and compared with that of insects on plants sprayed only with water and/or a formula blank.

Example 14

Treatment of Algal Infestations of Turfgrass

Algal infestation of turfgrass is most common on intensly managed turf (e.g., golf courses). Control of algal infestation has become difficult. Experiments to test for control of algae infestation are conducted using algal infested trufgrass plots. Infested turfgrass is treated with five test formulations and a formula blank, with five replications. Treatment effects are evaluated at two, three and five weeks.

Example 15

Treatment of Melon Aphid with Cinnamic Aldehyde

Plant Foliar Bioassay Treatment of melon aphid (*Aphis gossypii Glover*) was conducted as follows. Chrysantemums (*C. morifolum*) were used for melon aphid plant foliar bioassays. Plants were grown in 7.5 mm pots in planting soil in greenhouse. Flowering plants were infested and pre-count population size for each plant were taken and number of mean of aphids nymphs per leaf calculated. The plants were sprayed to runoff (about 5 ml) with an aqueous formulation containing 1,000 ppm, 3,000 ppm, and 10,000 ppm concentration of cinnamic aldehyde, and a negative control containing only $H_2O$. After 36 hours, the number of insects on the leaves sprayed with a given test formulation was determined and compared with that of insects on leaves sprayed with negative control only. Mean number of aphid nymphs per leaf were determined to be less than 10 for each cinnamic aldehyde concentration as compared to a pre-count mean of about 60. *See* Table 13.

Example 16

Treatment of Late Blight (*Phytophthora infestans*)

Late blight affects tomato, potato, eggplant and other potato family plants: it begins when fungal spores settle on wet plant surfaces during periods of mild temperature. Experiments to test for control of *Phytophthora infestans* are conducted using potato seedlings in the greenhouse. Plants are spray-inoculated with an isolate of the pathogen in the greenhouse. Plants are treated either prior to or after the inoculation. Treatment effects are evaluated at two and three weeks.

Table 13

| Treatment of melon aphid with cinnamic aldehyde | |
|---|---|
| Formulation | Mean Number of Aphid Nymphs Per Leaf |
| CNMA (ppm) | |
| 1,000 | 6 ± 3 |
| 3,000 | 4 ± 3 |
| 10,000 | 1 ± 1 |
| Control | |
| $H_2O$ | 33 ± 11 |
| Pre-count | 60 |
| *CNMA = cinnamic aldehyde (ppm) in $H_2O$. | |

Example 17

Treatment of *Vespidae*

To determine the contact activity of the formulas, test insects are directly sprayed. The treatment insects are removed and placed in untreated petri dishes or vials. Five formulas and a formula blank are tested, with five replicates for each formula and insect. The number of dead insects is counted at 24 and 48 hours.

Example 18

Treatment of *Dermoptera* - European Earwigs (*Forticula aureculatia*)

To determine the contact activity of the formulas, test earwigs are directly sprayed. The treated insects are removed and placed in untreated petri dishes or vials. Five formulas, a formula blank, and a negative control (water) are used for testing, with five replicates for each formula. The number of dead earwigs is counted at 24 and 48 hours.

Example 19

Residual Activity of Cinnamaldehyde and $\alpha$-hexyl Cinnamaldehyde

Two separate experiments indicated that both cinnamaldehyde (CNMA) and a-hexyl cinnamaldehyde (HCA) have residual activity. In the first experiment, two ml of two concentrations of CNMA (0.3 and 1%) were sprayed on filter paper (Whatman). As a negative control, two ml of water also were sprayed on filter paper. Twenty-four hours later, two ml of water were sprayed on treatment and control filter paper, which were then dried for 30 min. Approximately 30 thrips insects (*Frankliniella occidentalis*) were introduced and the number of *F. occidentalis* were observed after one hour. Mean mortality was calculated for each treatment.

After 72 hours, the treatment filter papers were flipped over and only the negative control filter paper and the filter paper treated with 1% CNMA were sprayed with 2 ml of water and allowed to dry for 30 minutes. Approximately 30 thrips were introduced to the two treated filter papers and after one hour the number of dead *F. occidentalis* were observed and the mean mortality calculated for each treatment. A similar assay was conducted using a-hexyl cinnamaldehyde. Mean mortality was higher for rehydrated filter papers compared to non-rehydrated filter papers over time. These experiments demonstrate that rehydration of the aldehyde residue plays a role in the continued lethal effects of treated filter paper in contact with thrips.

Continuous Exposure Tests To further determine the residual activity of CNMA and HCA, insects are confined to deposits on two representative surfaces. Glass is used to represent nonporous surfaces and filter paper is used as a porous surface. Two ml of five different concentrations of each active ingredient in a formula are applied to filter paper disks (9 cm diameter) or the bottoms of glass petri dishes (9 cm diameter). As a control, two ml of formula minus active ingredient are also applied. The deposits are allowed to dry for 24 hours before testing. At test intervals of 7, 14, 28, and 56 days, one set of plates and filter papers are rehydrated with 2 ml of water, while a parallel set is not rehydrated. Insects are then confined to the deposits continuously and the number of insects killed by the deposits is counted regularly. If deposits fail to kill insects within 48 hours, these treatments are discontinued from further aging studies.

Example 20

Western Flower Thrips

Dose-mortality response of CNMA formula against western flower thrips, *Frankliniella occidentalis,* was evaluated. Three different dose levels of the CNMA formulation were tested in comparison with a water only control. Tests were conducted using 500 ml ventilated paper cartons as experimental areas containing miniature rose foliage with approximately 30 adult *F. occidentalis* in each. An F. *occidentalis* colony (U.C. Davis, Department of Entomology) maintained on chrysanthemums and miniature rose was the source of thrips for the study. Three active ingredient dose levels (0.1,0.3, and 1.0%) were sprayed onto the plants using a laboratory spray tower calibrated to spray the field equivalent of 113 L per acre at the desired dosage. Four replicates (4 observations per treatment) were compared for each dosage. For each test, treatment cartons received the treatment and were maintained at room temperature. The mean mortality of *F. occidentalis* was observed for each treatment over time and mean mortality calculated. Table 14 presents the mean mortality treatment data.

Example 21

Treatment of Northern Corn Rootworm (*Diabrotica Longicornis*/*D. virgifaris*)

These corn root worms are among the most destructive insect pests of corn in North America. Greenhouse corn plants in 10 cm diameter pots from the University of Nebraska are infested with corn root worm prior to treatment. At the time of spraying, plant pots are placed in plastic bags so that chemical runoff from the leaf spray will not drip into the soil. Spray is applied to runoff with a household 1 liter capacity spray bottle. Plant are allowed to air dry outside then returned to the greenhouse. At weekly intervals thereafter, roots are separated from the potting mix and total root worm populations estimated.

Table 14:

| Effect of cinnamaldehyde (CNMA) concentration on thrip mortality over time1 | | | | |
|---|---|---|---|---|
| Time[2] | Mean Thrip Mortality | | | |
| | A | B | C | D[3] |
| 1615 | 0.79 | 0.26 | 0.13 | 0 |
| 1645 | 0.82 | 0.28 | 0.17 | 0 |
| 1715 | 0.78 | 0.28 | 0,17 | 0 |
| 1845 | 0.84 | 0.37 | 0.19 | 0 |
| 2215 | 0.84 | 0.40 | 0.25 | 0 |

1/ Whatmans paper plus rose leaf were added to small containers. 2 ml per treatment. Waited 5 minutes before addition of live trips.

2/ Recorded sampling time.

3/ A = 1.0% CNMA
B = 0.3% CNMA
C = 0.1% CNMA
D = Control (water only)

Example 22

Control of Boll Weevil (*Anthonomus grandis Boheman*)

To determine the contact activity of the formulae. cotton boll weevils are sprayed directly. The treated insects are removed and placed in sterile untreated petri dishes or vials. Five formulae and a formula blank are used in testing treatments, with five replicates tested for each formulae. The number of dead boll weevils is counted at 24 and 48 hours.

Example 23

Postharvest Treatment of Cut Flowers

Treatment of Cut Flowers for Vase Life Extension

Efficacy of two concentrations of four formulations of cinnamic and a-hexyl cinnamic aldehydes with surfactants is evaluated for extension of post harvest cut flower vase life. Postharvest control of bacterial and physiological plugging is tested on cut roses. Fifty (50) fresh harvested flowers are assigned to treatment and control (negative and positive) groups. Negative controls are deionized water and the positive control is OasisÔ floral preservative used per label. Individual flowers, treated and untreated, are placed in a Platex 240 ml baby bottle with a sterile bottle liner. Each bottle is graded over three week period for flower quality: straightness of stems, stem strength, flower size, vase life, maturity uniformity. All grading is by industry accepted standards.

Postharvest Control of *Botrytis cinerea* on Cut Flowers

*Botrytis* blossom blight, caused by *Botrytis cinerea* Pers: Fr., is a widespread and destructive disease on greenhouse-grown roses and many other cut flower crops. as well as grapes. Rose flowers (*Rosa Hybrida L.*) are immersed in the treatment solutions for 2-3 sec., then gently shaken to remove excess solution and allowed to dry. The flowers are then sprayed to runoff with a conidial suspension (*B. cinerea*) with a chromist spray unit (Gelman Sciences, Ann Arbor, MI). Non-inoculated flowers are sprayed with deionized water to monitor natural infection. After inoculation, the roses are placed in a humidified storage chamber at 2°C. Roses are removed from storage 7 days after inoculation, and disease development is quantified as the number of lesions on each flower.

Subsequently, the roses are evaluated for 10 days in a simulated consumer environment at 21°C with a 12-hour photo period from cool-white fluorescent lamps (PAR=32m $E.M^{-2}.S^{-1}$) (vase life evaluation). Fresh weights and visual observation are recorded daily. Roses are discarded during the 10 day period if *B. Cinerea* macerates the entire receptacle causing it to fall off the stem, or induces petal abscission.

Example 24

Control of Codling Moth, *Cydia pomonella*:

Susceptibility of Different Codling Moth Life Stages to Aromatic Aldehydes

Three life stages of codling moth are potentially exposed through contact or topical treatment to insecticide in the field: adults, eggs and neonate larvae before they enter the fruit. For optimal timing of field applications a susceptibility profile of these life stages is developed.

Susceptibility of Eggs

Residual toxicity: Strips of adhesive plastic foil (5 x 10 cm) are treated in a Potter spray tower with different concentrations of aromatic aldehyde formulae. After residue has dried the treated plastic strips are exposed to 10-15 moth pairs inside a cage for oviposition. After 24 to 48 hours, strips with eggs are removed, kept at 25°C and 60-70% relative humidity, and evaluated for egg mortality after eggs have hatched in the untreated control. This test also can be conducted with natural substrate to determine toxicity on fruit (apple) or leaves.

Topical toxicity: Eggs laid on plastic strips or fruit (apple) are treated in the Potter spray tower with different concentrations of the aromatic aldehyde formulae. Egg mortality is evaluated as above. Tests are conducted with young eggs (white stage) and eggs close to hatching (blackhead stage).

Susceptibility of Neonate Larvae: A larval assay described by Riedl *et al*. ((1986) *Agric. Ecosyst. Environ*. 16: 189-202) *is* used. Apples are treated in the Potter spray tower with different concentrations of the aromatic aldehyde formulae. Small gelatine capsules are attached with beeswax to the treated fruit surface. A single neonate larva is then placed inside a gelatine capsule. Apples with the caged larvae are kept at 25°C and 60-70% relative humidity. After 7 days, cages are removed to evaluate larval mortality and damage to the fruit (entries, stings).

Contact activity to neonate larvae also can be tested using a plastic petri dish assay. The interior surfaces of small petri dishes are treated in the spray tower. Neonates are exposed to the residue for various durations and then transferred to cups with untreated artificial diet. Mortality is assessed after ten days.

Toxicity and Sublethal Effects on Adults: Adults anesthetized with $CO_2$ are treated in the spray tower with different concentrations of aldehyde. Pairs of treated moths are placed in small oviposition cages. Development of

eggs from treated and untreated adults is compared. In addition, adult mortality is observed every 24 hours in treated and untreated adults until all moths have died.

Example 25

Control of San José Scale

To determine the contact activity of the aromatic aldehyde formulae, test scales are sprayed directly. The treated insects are removed and placed in sterile untreated petri dishes or vials. Five formula concentrations and a formula blank are used in testing treatments, which five replicates for each. The number of dead insects is counted at 24 and 48 hours.

Example 26

Control of Mealybugs

To determine the contact activity of the aromatic aldehyde formulae, test mealybugs are sprayed directly. The treated insects are removed and placed in sterile untreated petri dishes or vials. Five formula concentrations and a formula blank are used in testing treatments, with five replicates tested with each formula. The number of dead insects is counted at 24 and 48 hours.

Example 27

Phytotoxicity and Bioassay of Formulations Containing Cinnamic Aldehyde and Saponin

**A. Phytotoxicity Trials**

Phytotoxicity trials were performed on three greenhouse crops to determine the compatibility of using Saponin as surfactant adjuvant with CNMA in place of polysorbates (*e.g.* Tween). The following summarizes assay results:

1. Mini roses, (Sunburst). Four potted minirose plants (Sunburst) were treated with each of three treatment applications; 0.5% CNMA plus 0.05% Saponin, 0.25% CNMA plus 0.025% Saponin and a water only control. Plants were sprayed in the laboratory using a spray tower, all plants were sprayed to runoff. After spraying, plants were observed for a period of five days. No phytotoxicity was observed on old growth, new growth or on flower petals, indicating these rates are safe for applying to miniroses.

2. Chrysanthemums. Three potted chrysanthemums each were treated with 0.5% CNMA plus 0.05% Saponin, 0.25% CNMA plus 0.025% Saponin, or a water only control. Plants were sprayed as discussed above. After a five-day observation period no phytotoxicity was observed on leaf or flower petals, demonstrating these rates are safe for application.

3. Poinsettias. Two potted poinsettias each were treated with 0.5% CNMA plus 0.05% Saponin, 0.25% CNMA plus 0.025% Saponin, or a water only control. After a five-day observation period, phytotoxicity was observed on new leaf growth of the high application rate (0.5% CNMA, 0.05% Saponin). No symptoms were observed on new leaf growth of the lower rate (0.25% CNMA, 0.025% Saponin), indicating the lower rate is safe for application.

**B. Pest Insect Bioassays**

1. Two-spotted Spider Mites. Mites were assayed by placing rose leaves infested with spider mites in approximately equal numbers in petri dishes with Whatman paper placed on the bottom. Four petri dishes with mites were sprayed on both sides of leaves for each of three treatments: 0.5% CNMA plus 0.05% Saponin, 0.25% CNMA plus 0.025% Saponin and a water only control. Treated mites were left for 24 hours and the number of surviving mites were then counted and recorded. Results were as follows: Control petri dishes ($H_2O$ only), 53.25 ± 15.57 (mean±SE); 0.25% CNMA plus 0.025% Saponin, 6.75 ± 1.18; 0.5% CNMA plus 0.05% Saponin, 0.75 ± 0.48. These results indicate that CNMA plus Saponin have a high degree of efficacy against mites for direct spray applications.

2. Western Flower Thrips. Thrips were assayed by placing rose leaves infested with thrips in approximately equal numbers in petri dishes with Whatman paper placed on the bottom. Four petri dishes with thrips were sprayed on both sides of leaves for each of three treatments: 0.5% CNMA plus 0.05% Saponin, 0.25% CNMA plus 0.025% Saponin and a water only control. Treated thrips were left for 6 hours and the number of dead thrips were then counted and recorded. Results were as follows: Control petri dishes ($H_2O$ only), 1.4% ± 0.85% (mean SE); 0.25% CNMA plus Saponin, 53.2% ± 11.8; and 0.5% CNMA plus Saponin, 87.2% ± 2.79. These results indicate that CNMA plus Saponin have a high degree of efficacy against thrips for direct spray applications.

3. Melon Aphids. Melon aphids were assayed using whole, nonflowering potted chrysanthemum plants. Two plants were treated for each treatment and two leaves, one from the top of the plant and one from the bottom of the plant, were sampled to determine the number of living and dead melon aphids. Three treatments were applied: 1.0% CNMA plus 0.5% Saponin, 0.5% CNMA plus 0.25% Saponin, and 0.5% Saponin only. The whole plants were sprayed to "drip" on both the top and bottom sides of leaves. Results are presented as the proportion of aphids found dead. Results were as follows: control plant (0.5% Saponin only) 14.8% ± 4.5; 0.5% CNMA plus Saponin 48.3 ±16.1; 1.0% CNMA plus Saponin 72.0% ± 11.2. These results indicate the CNMA can kill a high degree of aphids with direct applications.

Example 28

Control of *Sclerotinia* dollar spot on turfgrass

Materials and Methods

Disease severity data was determined on a nursery green at Texas Agricultural Experiment Station-Dallas, Texas. The bentgrass green was composed of a sand/peat mixture (90:10). The green was maintained at a 0.4 cm cutting height with moderate fertilization and daily irrigation. The inoculum (SH-03) was prepared by growing a virulent isolate of S. *homoecarpa* on autoclaved rye grain for about 2 weeks prior to field inoculation. Plots were arranged in a randomized block design with three replications 2.5X18ft ($45ft^2$) in dimension. The infected rye grain was applied by hand-scattering at an approximate density of $20/ft^2$. Field applications of cinnamaldehyde formulations A, B, and C were applied at weekly intervals for four weeks (Table 15). Formulation D, however, was applied only one time due to phytotoxic reactions of the turfgrass. Formulations A, B, C and D were applied 4-days prior to field inoculation of the infected rye-grains, using a pressurized $CO_2$ sprayer (30 psi) at a volume of 7 gal/$1000ft^2$.

The experimental area was thoroughly watered following inoculation and mowing was suspended to allow fungal colonization. The inoculated area on each replication was covered with a plastic paper plate to insure high humidity for increased disease development. Disease assessment was made 2-days after inoculation by visual evaluation of the fungal mycelium growth from the infected rye-grain (0-4 max). A visual rating of the overall field plot appearance was also taken weekly, beginning three and a half weeks after the initial application and continuing for four days after the final application. This rating (0=phytotoxicity; 4=no disease) accounted for natural disease occurrence within the plots, also phytotoxicity caused by any formula application; 0=phytotoxicity, 1=heavy disease, 2=moderate disease, 3=slight disease, 4=no disease. Data were subjected to ANOVA using SAS ANOVA procedure to evaluate the statistical significance of treatment means (1). Where differences were detected, the Duncan multiple range comparison test (p>.05) was employed to separate treatment means.

Table 15

| Formulation Turf Kev | | |
|---|---|---|
| | CNMA[1] | T80[2] |
| A | 1.0 | 0.2 |
| B | 0.5 | 0.1 |
| C | 0.1 | 0.1 |
| D | 2.0 | 0.3 |

[1]CNMA = cinnamic aldehyde (%).
[2]T80 = Tween 80 (%).

Results

Two different methods were used to evaluate treatment plots for dollar spot disease suppression. The evaluation methods included 1) a natural disease outbreak method, and 2) a field inoculation method. The field inoculation technique is considered to be a severe form of disease pressure. The field inoculations were performed to approximate natural conditions for disease, with consideration given to natural inoculum loads for infection that occur with natural disease outbreaks.

The field inoculation method used evaluation of the fungicide Daconil 2787. The method was used in daily inoculations for 10 days and permitted detection of the fungicide for eight days after fungicide application. Experiments of this nature permitted determination of how quickly or slowly fungicide spray programs are negated by weather conditions, and cultural variables such as fertility levels, and greens cutting pressures, including cutting height and frequency, and the like.

Disease control with all test formulations (A, B, C and D) was observed for both evaluation methods as compared to untreated control areas.

<u>Control of natural outbreak of dollarspot:</u>

Dollarspot disease control was observed for all tested formulations in the test plots evaluated for natural disease suppression. Heavy disease pressure was observed on the untreated control plots throughout the experimental evaluation period. Treatment with cinnamaldehyde formulation D gave an initial phytotoxic reaction evidenced by symptoms of subtle yellowing of the entire leaf blade that appeared within 48 hours of treatment applications. Disease control by test formulations cinnamaldehyde formulations A, B, C, and D was better than the untreated control plots on each of the evaluation dates.

<u>Control of dollarspot following artificial inoculation:</u>

Dollarspot disease control was noted for all of the test cinnamaldehyde formulations evaluated for the artificially inoculated test plots (See Table 16). The untreated inoculated control plots gave severe disease ratings throughout the experiment with the mean mycelial outgrowth from the point of inoculation at 2.0 cm diameter or more 48 hours after inoculation. Cinnamaldehyde formulation D demonstrated dollarspot disease control, but its application was discontinued to avoid any permanent phytotoxicity damage to experimental bentgrass green. Surprisingly, results evaluated after 8 November were influenced by a shift to colder weather. Statistical separation of 32 treatments for the weekly mean disease ratings indicated that test formulations A, B and C were superior to the untreated control and had less disease for the non-cold weather dates. Formulation A, B and C treatments were all statistically the same on all four of the dates of observation.

The above results show that test formulations A, B, and C were effective against dollar spot foliar blighting under field plot inoculation conditions and where natural outbreaks of disease were present. Additional observations on the control of natural infestations of dollar spot also evidenced disease control by all four test formulations A, B, C and D. The results were surprising given the level and duration of dollar spot disease control by the cinnamaldehyde formulations.

Table 16

| Formulation turf application interval and disease control assessment for *Sclerotinia* dollarspot disease on a "Penncross" bentgrass green | | | | | | |
|---|---|---|---|---|---|---|
| | | Average Disease Index (0-4 max)[d] | | | | |
| Formulation[a,b] 7 gal/ $1000ft^2$ | Interval[c] Application (Days) | Week 1 | Week 2 | Week 3 | Week 4 | |
| A | Weekly | 1.9 | | 2.1 | 1.3 | 3.0 |
| B | Weekly | 1.0 | | 2.3 | 1.4 | 3.0 |
| C | Weekly | 2.0 | | 2.5 | 1.4 | 3.3 |
| D | 1X | ----- | | 2.7 | ----- | ----- |
| Untreated | | 3.0 | | 3.5 | 1.4 | 3.5 |

a/ Sprays were applied weekly using a hand-held boom using $CO_2$ pressurized sprayer 30 psi.

b/ See Table 15 for formulation information

c/ All applications were applied weekly except for Formulation D, which was applied only 1x due to turfgrass phytotoxic reactions.

d/ Disease assessments were rated on a visible scale of 0-4; 0=no disease, 1=fungal growth initiated, 2=fungal growth 0.5cm, 3=fungal outgrowth 1 cm, and 4=fungal outgrowth 1cm.

Example 29

Control of *Pvthium* blight on turfgrass

Disease severity data was determined using environmental chamber studies, which were conducted using turfgrass plugs (7 cm diam) taken from a bentgrass green located at Texas Agricultural Experiment Station-Dallas, Texas. The bentgrass green was composed of a sand/peat mixture (90:10) and was maintained at 0.4 cm cutting height with moderate fertilization and daily irrigation. Plots were arranged in a randomized block design with 3 replications 2.5 x 13 ft (32.5 $ft^2$) in dimension. Cinnamaldehyde formulations A, B, C and D (Table 15) and the positive control AleietteÔ (4 oz) were applied 4 days prior to environmental chamber inoculations with field plugs (7 cm diam.). Formulations were applied using a pressurized $CO_2$ sprayer (30 psi) with a volume of 7 gal/1000 $ft^2$ at rates according to the manufacturer's specifications.

The inoculum P#24 was grown on sterile water agar for 3 days for the environmental chamber studies. Environmental chamber studies used 3 replications of bentgrass plugs inoculated with the pathogen P#24 grown on water agar. Each bentgrass plug was inoculated in the center with one I cm plug of water agar containing the pathogen. The bentgrass plugs were placed in a lighted walk-in environmental chamber maintained at 28°C for 4 days prior to disease assessment. The plugs were watered daily to insure a high humidity environment for optimal disease pressure. Disease assessment for the environmental chamber study was made after 4 days with disease spread determined by mycelium spread (cm diameter).

Data were subjected to ANOVA using SAS ANOVA procedure to evaluate the statistical significance of the treatment means. Where significance differences were detected, the Duncan multiple range comparison test ($p>0.05$) was employed to separate treatment means. Each of cinnamaldehyde formulations A, B, C, and D controlled *Pythium* blight disease in the environmental chamber (Table 17). In contrast, the untreated, inoculated controls had severe disease ratings throughout the experiment, with the mean mycelial outgrowth from the point of inoculation at 5.0 cm or more 48 hours after inoculation (Table 17). A single application of cinnamaldehyde formulation D showed phytotoxicity on treated bentgrass with symptoms of foliar yellowing and stunted growth and was applied only once.

The above results show that cinnamaldehyde formulations A, B and C were effective for four weeks after treatment. These results were surprising given the level and duration of disease control, particularly when compared to the standard fungicide Aliette 4.0 oz, which was sprayed weekly over the four week study period.

Table 17

Formulation turf application interval and disease control assement for *Pythium* blight in environmental chamber inoculations of "Penncross" bentgrass green

| | | Disease Spread (cm)[d] | | | |
|---|---|---|---|---|---|
| Formulation[a,b] 7 gal/ 1000$ft^2$ | Interval[c] Application (Days) | Week 1 | Week 2 | Week 3 | Week 4 |
| A | Weekly | 3.8 | 4.3 | 4.7 | 4.6 |
| B | Weekly | 4.5 | 5.0 | 5.0 | 4.6 |
| C | Weekly | 4.0 | 3.8 | 4.0 | 3.6 |
| D | 1X | ----- | 5.5 | ----- | ----- |
| Aliette 4.0 oz | Weekly | 4.0 | 4.2 | 4.8 | 4.8 |
| Untreated | | 5.9 | 4.9 | 6.5 | 6.1 |

a/ Sprays were applied with a hand-held boom using a $CO_2$ pressurized sprayer (30 psi).

b/ See Table 15 for formulation components

c/ All applications were applied weekly except for ProGuard D, which was applied only 1x due to turfgrass phytotoxic reactions.

d/ Disease spread were measured in cm. across turfgrass plugs 7 cm diam in size; 0=no disease, 1=fungal growth initiated, 2=fungal growth 0.5cm, 3=fungal outgrowth 1 cm, and 4=fungal outgrowth > 1cm.

Example 30

Control of *Rhizoctonia* blight on turfgrass

Disease severity data was determined on a nursery green at Texas Agricultural Experiment Station-Dallas, Texas. The bentgrass green was composed of a sand/peat mixture (90:10). The green was maintained at a 0.4 cm cutting height with moderate fertilization and daily irrigation. The inoculum (R#31) was prepared by growing a virulent isolate of *Rhizoctonia solani* on autoclaved rye grain for about 5 days prior to field inoculation. Plots were arranged in

a randomized block design with three replications 2.5X18ft ($45ft^2$) in dimension. The infected rye grain was applied by hand-scattering at an approximate density of 20 grains/$ft^2$. Field applications of each fungicide were initiated and continued for three weeks. Spray intervals for the Daconil 6.0 oz positive control was according to the manufacturers specification. The cinnamaldehyde A, B, C and D formulations (See Table 15) were applied weekly (D only once), while Daconil 6.0 oz. was applied every 14 days. All formulations were applied 4-days prior to field inoculation of the infected rye-grains. Formulations were applied using a pressurized $CO_2$ sprayer (30 psi) at a rate of 7gal/1000$ft^2$. The experimental area was thoroughly watered following inoculation and mowing is suspended to allow fungal colonization. The inoculated area on each replication was covered with a plastic paper plate to insure high humidity for increased disease development.

Disease assessment was made 2-days after inoculation by visual evaluation of the fungal mycelium growth from the infected rye-grain (0.4 max); 0=no disease, 1 mycelium growth initiated 2=.5 cm growth, 3=1cm growth, and 4=>1 cm growth. Data were subjected to ANOVA using SAS ANOVA procedure to evaluate the statistical significance of treatment means. Where differences were detected, the Duncan multiple range comparison test (p>0.05) was employed to separate treatment means.

Control of *Rhizoctonia* blight disease was noted with all of the test cinnamaldehyde formulations in the inoculated test plots evaluated for disease suppression (See Table 18). The untreated inoculated controls gave severe disease ratings throughout the experiment with the mean mycelial outgrowth from the point of inoculation at 2.8 cm diameter or more 48 hours after inoculation. Phytotoxicity was observed following a single application of formulation D, and thus its application was discontinued.

The above results demonstrate that formulations B and C were not phytotoxic and were comparable to Daconil 6 oz for the control of *Rhizoctonia* blight of bentgrass in inoculation field trials. These results were surprising given the level and duration of disease control observed for formulations B and C, which was at least equal to Daconil 6.0 oz.

Table 18

| Formulation turf application interval and disease control assessment for Rhizoctonia blight on "Penncross" bentgrass | | | | | |
|---|---|---|---|---|---|
| | | Average Disease Index (0-4 max)[d] | | | |
| Formulation[a,b] 7 gal/ 1000$ft^2$ | Interval[c] Application (Days) | Week 1 | Week 2 | Week 3 | Week 4 |
| Daconil 6oz | 14 Days | 1.3 | 1.8 | 1.9 | 2.0 |
| A | 7 Days | 1.2 | 2.9 | 2.8 | 2.4 |
| B | 7 Days | 1.5 | 2.5 | 1.8 | 2.1 |
| C | 7 Days | 1.5 | 2.6 | 2.0 | 2.5 |
| D | 7 Days* | | 3.0 | 2.2 | 2.4 |
| Inoculated Check | | 2.8 | 3.1 | 3.0 | 3.0 |

a/ Sprays were applied with a hand-held boom using a $CO_2$ pressurized sprayer (30 psi).

b/ See Table 15 for components of each formulation

c/ Applications at 7 days were applied 4 times at 7 day intervals. Applications at 14 days were applied twice at 14 day intervals, * indicates treatment was phytotoxic to turfgrass and applied only once.

d/ Disease assessments were rated on a visible scale of 0-4. 0=no disease; 1=fungal outgrowth initiated; 2=fungal outgrowth 0.5 cm; 3=fungal outgrowth 1cm; 4=fungal outgrowth > 1cm.

Example 31

Overproduction of Aromatic Aldehydes in Transgenic Plants

Twenty mg of polyA RNA is prepared from a plant tissue that produces cinnamaldehyde, and cDNA is synthesized. Part of this is cloned into lambda-ZAP II vector (a commercially available cloning vector). At least 500,000 recombinants are screened using an oligonucleotide probe designed from conserved sequences of cloned CA4H and CAD genes obtained from GenBank, or designed from peptide sequence of purified protein from the intended host plant. Strongly hybridizing clones are selected and used to rescreen the cDNA library. The resulting clones are sequenced to enable the introduction of appropriate gene sequences into a plant expression cassette in either antisense or sense orientation. The antisense and sense constructs are introduced into *Agrobacterium tumefaciens* LBA4404 by direct transformation following published procedures. Tobacco *(N. tabacum* variety Samsun) leaf discs are transformed using well estabished published procedures (Horsch *et al.* (1985) *Science* 227:1229-1231. Plants containing either CA4H or CAD constructs are identified by PCR or hybridization and selected for further analysis.

Plant material from both transformed and untransformed control plants is used for determinations of CA4H

and CAD enzyme activity using well established published assays. Plants in which the activity of CA4H or CAD has been reduced to less than 20% of that seen in control plants are selected for further analysis. Selected plants with low CA4H activity are crossed with plants with low CAD activity and progeny inheriting both gene constructs are selected by PCR. Plants with suppressed CA4H and suppressed CAD activity are analyzed for aromatic aldehyde production using standard published procedures.

Example 32

Production of Aromatic Aldehydes in Microbial Systems

A cDNA library is generated using RNA extracted from six week old tobacco stems. 20mg of polyA RNA is prepared and cDNA synthesized. Part of this is cloned into lambda-ZAP II vector (a commercially available cloning vector). At least 500,000 recombinants are screened using an oligonucleotide probe designed from peptide sequence sequences of CCoAr protein purified from six week old tobacco stem tissue using the protocol of Goffner, et al., *Plant Physiol*. (1994) 106:625. Strongly hybridizing clones are selected and used to rescreen the cDNA library. The resulting clones are sequenced to enable the identification of full-length cDNA inserts and the introduction of appropriate CCoAR gene sequences into yeast expression vector pMTL8110 (Faulkner, et al (1994) *Gene* 143:13-20. The coding sequences for *Rhodosporidium toruloides* phenylalanine ammonia lyase (PAL; GenBank locus RHDPAL) and a parsley 4-coumarate:CoAl ligase (4CL; GenBank locus PC4CL1AA) are similarly introduced into equivalent yeast expression vectors. The PAL,4CL and CCoAR constructs are used to transform *Saccharomyces cerevisiae* strains by electroporation using established published procedures (Becker, and Guarente, *Methods in Enzymology* 194:182-187, 1991; Simon, (1993) *Methods in Enzymol* 217:478-483. Transformants are selected on minimal medium lacking leucine. Transformant strains carrying all three gene constructs are identified by PCR and selecter for further analysis.

Extracts from both transformed and untransformed control strains are used for determinations of PAL, 4CL and CCoAR enzyme activities using well established published assays, Strains in which the activity of PAL, 4CL and CCoAR is significantly greater than the background activity detected in control strains are selected for further analysis. Selected strains are analyzed for aromatic aldehyde production using standard published procedures and those producing significant amounts of cinnamaldehyde are selected for optimization of fermentation conditions.

Example 33

Construction of a Yeast Strain that Produces HCA

A yeast strain, such as *Saccharomyces cerevisiae*, which contains the enzymes necessary for biosynthesis of cinnamaldehyde (CNMA) is constructed as follows. First, the strain is engineered for high-level expression of PAL as described by Faulkener *et al*. (1994) *Gene* 143:13020. A plant cinnamate 4-hydroxylase gene is also operably linked to appropriate control signals and inserted into the strain (*see* Urban *et al.* (1994) *Eur. J. Biochem*. 222:843-850). A cinnamoylCoA reductase (CCoAR) gene is obtained by applying standard gene cloning techniques to isolate a cDNA clone using as a probe a nucleotide sequence derived from a partial amino acid sequence of the purified protein, which has been purified and partially characterized from several plant sources. The CCoAR gene is also linked to control signals operable in yeast and inserted into the yeast strain.

The gene encoding the enzyme that catalyzes the conversion of CNMA to HCA is then cloned as follows. A cDNA library is constructed in a yeast expression vector using mRNA obtained from rice plants. The cDNA library is then transformed into the previously constructed yeast strain and transformants selected using a selectable marker present on the expression vector.

To identify those yeast strains that produce HCA, transformants are transferred to microtiter wells containing yeast growth medium agar. Microtiter plates are then placed in a chamber that contains fleas, which are sensitive to HCA but not to CNMA. Yeast strains from wells that contain a statistically significantly greater number of dead fleas than wells containing untransformed control yeast strains are diluted and re-plated in microtiter plates, after which the screening process is repeated to obtain colonies derived from a single transformed yeast cell.

Single cell-derived colonies that exhibit increased flea mortality are analyzed for HCA production by gas liquid chromatography (GLC), using a 30 meter non-polar polydimethylsiloxane capillary column (*e.g.* HP-1, Hewlett-Packard, or SPB-1, Supelco) and a flame-ionization detector. Using helium as a carrier gas (8 ml/min.) and a column temperature of approximately 240°C, the (E)-cis isomer (major component) has a retention time of approximately 6.0 minutes and the (Z)-trans isomer (minor component) has a retention time of approximately 6.3 minutes.

Expression vector DNA is isolated from colonies that produce HCA and the insert sequenced to obtain the nucleotide sequence and deduced amino acid sequence of the enzyme that catalyzes the conversion of CNMA to HCA.

Example 34

Construction of Transgenic Plants that Produce HCA

A gene that codes for the enzyme that catalyzes the conversion of cinnamic aldehyde (CNMA) to a-hexyl cinnamic aldehyde (HCA) is cloned from rice plants by transposon mutagenesis. Rice protoplasts are transformed with a cloning vector that contains a maize Ac transposable element inserted into a hygromycin B phosphotransferase (*hyg*B) gene so as to disrupt the coding sequence of the hygB gene (*see,* e.g., Izawa *et al*. (1991) *Mol. Gen. Genet.* 227: 391-396; Murai *et al.* (1991) *Nucl. Acids. Res*. 19: 617-622). Transformed protoplasts are plated on growth medium agar containing sufficient amounts of hygromycin B to prevent non-hygromycin resistant protoplasts from regenerating. Protoplasts in which the Ac transposable element has jumped from the *hyg*B gene to the rice genome are resistant to hygromicin B, and thus regenerate to form callus tissue. Plants are regenerated from hygromycin-resistant callus tissue *(see,* Izawa *et al*., *supra.*).

Regenerated rice plants are analyzed for the presence or absence of CNMA and HCA as described in the previous Example. Plants that produce CNMA but not HCA potentially carry an AC transposon inserted into the HCA biosynthesis gene. Genomic DNA is isolated from $CNMA^+/HCA^-$ mutants and hybridized to labeled transposon DNA. Fragments that hybridize to the transposon DNA probe are subcloned into an appropriate cloning vector for mapping and sequence analysis. The HCA biosynthesis gene is identified as an open reading frame that is disrupted by insertion of the known sequence of the Ac transposon. A fragment of the gene is used to probe a cDNA library to isolate the corresponding cDNA.

The cDNA for the HCA biosynthesis enzyme is inserted into an expression vector, operably linked to a strongly expressed promoter that is functional in plant for which pest resistance is desired. The expression vector is inserted into the plants using transformation methods known to those of skill in the art. Transgenic plants are analyzed for HCA production and/or repellent or pesticidal activity against pests as described in Example 33 above.

Example 35

Evaluation of Storax Combined with Cinnamic Aldehyde or α-Hexyl Cinnamic Aldehyde Against Melon Aphid

In previous bioassays evaluating the efficacy of cinnamic aldehyde against melon aphid (*Aphis gossypli Glover*) on plants sprayed to run-off at 1,000 ppm, an $LD_{50}$ was observed at 2 hrs and an $LD_{75}$ at 24 hrs. The purpose of this study was to determine the effect of STORAX incorporated into various formulations to evaluate its potential use as a synergist. Initial bioassays were conducted using STORAX at 0.6%, 1.0%, 2.0% with Tween 80 (1%) only. The results are presented in Figure 1. A second bioassay was conducted to evaluate the efficacy of STORAX (at 0.6%) alone and STORAX (at 0.6%) plus cinnamic aldehyde (at 0.1%) or alpha hexyl cinnamaldehyde (at 0.1%). The results are presented in Figure 2. At 25 hours post treatment by water, Storax alone, Storax plus HCA and Storax plus CNMA, the percent mortality of melon aphids were 33, 74, 95.1 and 94.6 respectively. STORAX combined with cinnamic aldehyde or α-hexyl cinnamic aldehyde reduced the time course of lethality and increases mortality. Moreover, the cinnamic aldehyde-STORAX formulation approaches the lethal time (LT) required at 2h for kill of certain virus transmitting pests (e.g., brown citrus aphid). Observations indicate that STORAX inhibited phytotoxicity for foliar application at an aldehyde concentration <0.5% on sensitive plants (e.g., glasshouse rose varieties).

Example 36

Proposed Bioassay of Pesticide Efficacy Against Brown Aphid

Preliminary bioassays using the formulations listed below in Table 19have shown a high degree of efficacy against aphid populations such as the Melon aphid. The results thus far show that these materials can kill a high percentage of the aphid population in a relatively short time period (up to 95% in <3 hr at some concentrations). The following protocol is designed to evaluate the efficacy at the indicated formulations against the brown aphid and to estimate the lethal dosage (LD) and lethal time (LT) of the different treatment regimens on the brown aphid. The brown aphid infects citrus trees with the potent virus called tristeza. To be effective in the field, a significant degree of lethality (LD90+) is required within 2 h of treatment. The treatment regimen and percent by weight of the test compound are as shown in Table 19.

Table 19

| Test Formulations for Brown Aphid Treatment | |
|---|---|
| Treatment | CNMA or HCA (% by weight) |
| Water + Tween 80 (1.0%) | ----- |
| Storax (1.0%) + Tween 80 (1.0%) | ----- |
| CNMA + Tween 80 (1.0%) | 0.1, 0.25, 0.50 |
| Storax (1.0%) + CNMA + Tween 80 (1.0%) | 0.1, 0.25, 0.50 |
| Storax (1.0%) + HCA+ Tween 80 (1.0%) | 0.1, 0.25, 0.50 |
| Water only | ----- |

Trials are conducted using ≥ 4 replicates per treatment and approximately 50 or more aphids per replicate. This results in a total of 44 observations for the trial. Material is applied by foliar spray to run off at the prescribed concentrations by volume as presented in the treatment list above. The number of aphids killed for each treatment is recorded at 1 h, 2 h, 6 h, and 24 h. The LD is calculated from the total proportion of aphids killed for a given dosage of active ingredient in the formulations. LT is calculated by determining the elapsed time to reach a proportion killed at a given formula concentration.

Example 37

Bioassay of Pesticide Efficacy and Phytotoxicity

Preliminary bioassays using the active ingredients and formulations listed in the Table below have shown no observable phytotoxicity on subject plants. The following protocol is to conduct preliminary evaluation for phytotoxicity on roses of the indicated formulations (Table 20).

Table 20

| Test Formulations for Rose Treatment | |
|---|---|
| Treatment | CNMA or HCA |
| Water + Tween 80 (1.0%) | ----- |
| Storax (1.0%) + Tween 80 (1.0%) | ----- |
| CNMA + Tween 80 (1.0%) | 0.1, 0.25, 0.50 |
| Storax (1.0%) + CNMA + Tween 80 (1.0%) | 0.1, 025, 0.50. |
| Storax (1.0%) + HCA+ Tween 80 (1.0%) | 0.1, 0.25, 0.50 |
| Water only | ----- |

Phytotoxicity trials are conducted using a 4 by 3 design (4 repetitions with three observations per repetition per treatment). Tests compare the effect of 12 formulation treatments, 9 containing one or more active ingredients and 3 control treatments for comparison with respect to phytotoxicity symptoms. Materials are applied with a hydraulic sprayer to drip. Up to 3 treatment applications are made at 7 day intervals for each formulation. Phytotoxicity symptoms are assessed visually at 3 days post-application for each of the three applications.

Example 38

Treatment of 1$^{st}$ and 2$^{nd}$ Instars of Silverleaf Whitefly (Bemisia argentifolii) with Cinnamic aldehyde on Poinsettia (Euphorbia pulcherrima)

Bioassay

Clip cages were set on poinsettia plants leaves to capture white fly adults which then were allowed to oviposit for 48 hours. Four plants per treatment or control were put into an environmental chamber and the eggs allowed to incubate for 5 days until a majority of the eggs had hatched to first and second instar. Three treatments at 0.5% cinnamic aldehyde and 0.25% Tween 20 (T1, T2, T3) and three control treatments of 0.25% Tween 20 only (C1, C2, C3) were sprayed to run off on each plant. Mortality was recorded after 48 hours *(see* Table 21).

Table 21.

| Treatment of Silverleaf Whitefly Instars (1 and 2) | | | |
|---|---|---|---|
| | Dead | Alive | Mortality(%) |
| T1 | 51 | 5 | |
| T2 | 176 | 13 | |
| T3 | 136 | 17 | |
| | 363 | 35 | 91% |
| C1 | 31 | 33 | |
| C2 | 14 | 220 | |
| C3 | 6 | 262 | |
| | 51 | 515 | 9% |

Example 39

Treatment of Melon Aphid on Chrysanthemum with Cinnamic Aldehyde encapsulated in two different wax shells (beeswax and Carnauba wax)

Cinnamic aldehyde (1%) was sub-microencapsulated at the one micron size in a beeswa or carnauba wax solution. Treatment of melon aphid (*Aphis gossypii Glover*) was conducted as follows. Chrysanthemum (*C. morifolium*) leaves infested with melon aphid were selected at random and assigned to either a treatment (T1 or T2) or control block (C1 or C2). A total of 9 leaves, each leaf a replicate, was assigned to each treatment or control. Using a laboratory spray tower calibrated to spray the field equivalent of 113L per acre at the desired dosage, T1 and T2 and their control formula blanks, C1 and C2, were sprayed. After each treatment, the cartons were maintained at room temperature. The mortality of melon aphid was observed for each spray at 24 hours and mortality recorded. *(see* Table 22).

Table 22

| Treatment of melon aphid with microencapsulated cinnamic aldehyde | |
|---|---|
| Treatment | Melon Aphid *(Aphis gossypii Glover)* Mortality at 24 hrs. |
| T1 (1% Cinnamic Aldehyde carnauba wax shell) | 90%+ |
| T2 (1% Cinnamic Aldehyde beeswax shell) | 90%+ |
| C1 (Formula blank - carnauba) | 5% |
| C2 (Formula blank - beeswax) | 5% |

Example 40

Treatment of Spider Mites on Bean Leaf Disk.

α-hexylcinnamic aldehyde (HCA) at a concentration of 0.1, 0.3 and 1.0% was submicroencapsulated at the one micron size in beeswax or carnauba wax. Leaf disks (20 mm diameter) were cut from bean leaves and placed on moist cotton, then ten adult female mites were placed on each leaf disk and sprayed in a Potter spray tower with 2 ml of one of the treatment formulations. Control mites were treated with distilled water. A total of 30 mites (3 leaf disks) were treated with each concentration. Sprayed leaf disks were held at high humidity at 70°F in a growth chamber. Mortality was assessed 48 and 72 hours after mites were sprayed. The results are summarized in Table 23.

Table 23

| Treatment of Mites with Microencapsulated HCA | | | | |
|---|---|---|---|---|
| Treatment | Concentration % | No. Mites Treated | % Mortality | |
| | | | after 48h | after 72 h |
| HCA in camauba wax | 0.1 | 45 | 11.1 | 17.8 |
| | 0.3 | 55 | 41.8 | 52.7 |

Table 23 (continued)

| Treatment of Mites with Microencapsulated HCA | | | | |
|---|---|---|---|---|
| Treatment | Concentration % | No. Mites Treated | % Mortality | |
| | | | after 48h | after 72 h |
| | 1.0 | 55 | 100.0 | 100.0 |
| HCA in beeswax | 0.1 | 30 | 16.7 | 36.7 |
| | 0.3 | 30 | 27.5 | 40.0 |
| | 1.0 | 30 | 97.5 | 100.0 |
| Untreated control | | 55 | 12.8 | 12.8 |

Both formulations appeared to be equally effective. For both formulations, the dose response was between 0.1 and 1.0%. Intermediate concentrations between 1.0 and 0.3% need to be evaluated to better define the dose response lines. All mites treated with the 1.0% concentration died after 72 hours. Both formulations left a noticable white residue on the leaf surface.

Example 41

Treatment of Melon aphid on Chrysanthemum with 5% alpha Hexylcinnamic aldehyde in emulsion and microencapsulated

Treatment of Melon aphid (*Aphis gossypii Glover)* on chrysanthemum leaves was conducted to evaluate the efficacy of α-hexylcinnamic aldehyde in different formulations. Efficacy was compared among three treatments (5% HCA, 1% Tween 20 in water; 5% HCA microencapsulated in beeswax; and 5% HCA microencapsulated in carnauba wax) and a water only control. Three chrysanthemum leaves infested with melon aphids were used for each treatment. Four replicates (4 observations per treatment) were compared for each formulation. Tests were conducted using 500 ml ventilated paper cartons as experimental arenas containing approximately 50 adult *Aphis gossypii* in each per leaf (150 aphids per arena). For each test, treatment cartons received the active ingredient as a 5% aqueous emulsion or as microcapsules in either beeswax or carnauba wax. The negative control was a water only treatment. Sprays of each treatment were applied using a laboratory spray tower calibrated to spray the field equivalent of 113 liters per acre. The paper carton arenas were maintained at room temperature. Experimental cartons were examined at 24 hours post-treatment and % mortality determined *(see* Fig. 3). Every treatment regimen killed 100% of the melon aphids at 24 hours.

The above examples demonstrate that the subject aromatic aldehyde formulations and methods are useful for treatment and/or prevention of infestation of plants by a wide variety of pest organisms. The formulations are effective in treating roses for powdery mildew, rust, and aphid infestations and grapes for phylloxera infestation (with the formulations being effective at the egg, nymphal, and adult life stages of phylloxera). The formulations were also shown to be effective against pests such as spider mite, aphid, white fly, nematode, and thrips. The formulations also were effective against fungal pathogens such as *Fusarium subglutinans, Botrytis cinera*, and turfgrass pathogens such as *Sclerotinia* dollar spot, *Rhizoctonia* blight and *Pythium* blight.

## Claims

**1.** A method for providing a susceptible plant with increased resistance to pathological microorganisms, said method comprising:

providing said plant with a nonphytotoxic composition comprising at least one aromatic compound having formula

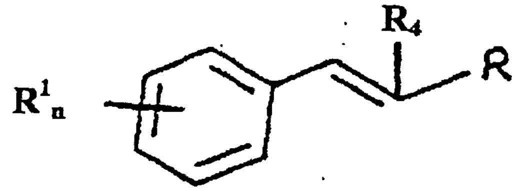

(1)

wherein R represents -CHO, $CH_2OH$, -COOH, or $-COOR_5$, n is an integer from 0 to 3; each $R^1$ represents -OH or an organic substituent containing from 1 to 10 carbon atoms and from 0 to 5 heteroatoms, wherein the total number of carbon and heteroatoms in all $R^1$ substituents of said compound is no more than 15; and $R_4$ represents -H or an organic substituent containing from 1 to 10 carbon atoms; and $R_5$ represents an organic substituent containing from 1 to 10 carbon atoms and from 0 to 5 heteroatoms which increases accumulation of aromatic aldehydes in said plant or increases cinnamic acid in said plant, wherein said composition does not include an additional anti-oxidant other than said aromatic compound, whereby at least one of growth and viability of a pathological microorganism which colonizes a surface or a part of said plant is impaired, wherein said aromatic aldehyde is microencapsulated in a polymer.

**2.** The method according to claim 1, wherein said polymer is beeswax or carnauba wax.

**3.** The method according to claim 1, wherein said agent comprises a balsam.

**4.** The method according to claim 3, wherein said balsam is derived from a *Liquidambar* tree.

**5.** The method according to claim 4, wherein said *Liquidambar* tree is *Liquidambar orientalis Miller* or *Liquidambar sytraciflua*.

**6.** The method according to claim 3, wherein said agent further comprises one or both of cinnamic aldehyde and alpha-hexyl cinnamic aldehyde.

**7.** A method for controlling growth of pathological organisms on a plant whereby the plant surface is provided with a nonphytotoxic composition comprising a balsam and one or more aromatic compounds having the formula

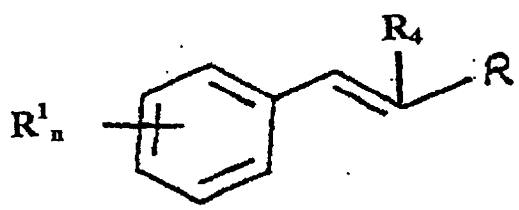

(1)

wherein R represents -CHO, $CH_2OH$, -COOH, or $-COOR_5$, n is an integer from 0 to 3; each $R^1$ represents -OH or an organic substituent containing from 1 to 10 carbon atoms and from 0 to 5 heteroatoms, wherein the total number of carbon and heteroatoms in all $R^1$ substituents of said compound is no more than 15; and $R_4$ represents -H or an organic substituent containing from 1 to 10 carbon atoms; and $R_5$ represents an organic substituent containing from 1 to 10 carbon atoms and from 0 to 5 heteroatoms which increases accumulation of aromatic aldehydes in said plant or increases cinnamic acid in said plant, wherein said composition does not include an additional anti-oxidant other than said aromatic compound, whereby at least one of growth and viability of a pathological microorganism which colonizes a surface or a part of said plant is impaired.

**8.** The method according to claim 7, wherein said pathological organisms are aphids.

**9.** The method according to claim 7 or 8, wherein said composition comprises a surfactant.

**10.** The method according to any one of claims 7-9 comprising one or more aromatic aldehydes having the formula

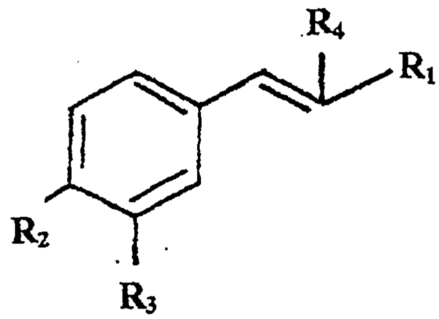

wherein $R_1$ represents -CHO, $R_2$ represents -H, -OH or an organic substituent containing from 1 to 10 carbon atoms, and $R_3$ represents -H, a methoxy group or organic substituent containing from 1 to 10 carbon atoms, and $R_4$ represents -H, or an organic substituent containing from 1 to 10 carbon atoms.

**11.** The method according to claim 10, wherein said aromatic aldehyde is selected from the group consisting of cinnamic aldehyde, alpha-hexyl cinnamic aldehyde and coniferyl aldehyde.

**12.** A composition comprising one or more aromatic aldehydes of formula

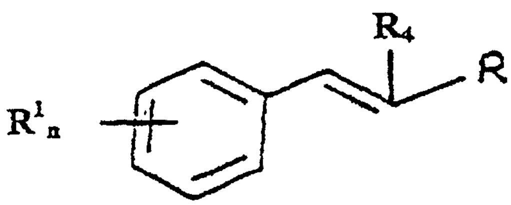

(I)

wherein R represents -CHO, $CH_2OH$, -COOH, or $-COOR_5$, n is an integer from 0 to 3; each $R^1$ represents -OH or an organic substituent containing from 1 to 10 carbon atoms and from 0 to 5 heteroatoms, wherein the total number of carbon and heteroatoms in all $R^1$ substituents of said compound is no more than 15; and $R_4$ represents -H or an organic substituent containing from 1 to 10 carbon atoms; and $R_5$ represents an organic substituent containing from 1 to 10 carbon atoms and from 0 to 5 heteroatoms which increases accumulation of aromatic aldehydes in said plant or increases cinnamic acid in said plant, wherein said composition does not include an additional antioxidant other than said aromatic compound, whereby at least one of growth and viability of a pathological microorganism which colonizes a surface or a part of said plant is impaired and said composition comprising a balsam in a formulation which is nonphytotoxic to plants, wherein the concentration of said balsam is sufficient to provide a mean disease control of 70%.

**13.** The composition according to claim 12, wherein said composition further comprises one or more aromatic aldehydes having the formula:

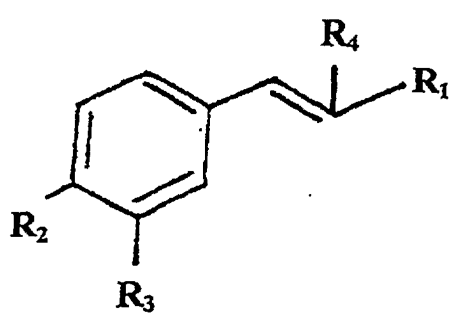

wherein $R_1$ represents -CHO, $R_2$ represents -H, -OH or an organic substituent containing from 1 to 10 carbon

atoms, and $R_3$ represents -H, a methoxy group or organic substituent containing from 1 to 10 carbon atoms, and $R_4$ represents -H, or an organic substituent containing from 1 to 10 carbon atoms.

**14.** The composition according to claim 13, wherein said aromatic aldehydes is selected from the group consisting of cinnamic aldehyde, alpha-hexyl cinnamic aldehyde and coniferyl aldehyde.

**15.** The composition according to claim 12, wherein said formulation is an emulsion.

**Patentansprüche**

**1.** Verfahren zum Erzeugen einer erhöhten Resistenz gegenüber pathologischen Mikroorganismen bei einer anfälligen Pflanze, wobei das Verfahren folgendes umfasst:

Behandeln der Pflanze mit einer nichtphytotoxischen Zusammensetzung, die wenigstens eine aromatische Verbindung mit der folgenden Formel umfasst:

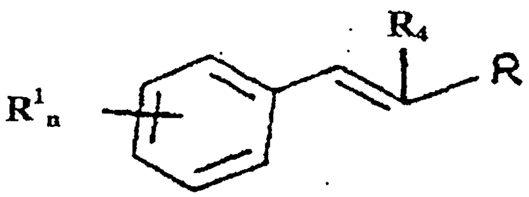

(I)

wobei R -CHO, $CH_2OH$, -COOH oder -$COOR_5$ darstellt, n eine ganze Zahl von 0 bis 3 ist, jedes $R^1$ -OH oder einen organischen Substituenten mit 1 bis 10 Kohlenstoffatomen und 0 bis 5 Heteroatomen darstellt, wobei die Gesamtzahl an Kohlenstoff- und Heteroatomen in allen $R^1$-Substituenten der Verbindung nicht größer als 15 ist, und $R_4$ -H oder einen organischen Substituenten mit 1 bis 10 Kohlenstoffatomen darstellt und $R_5$ einen organischen Substituenten mit 1 bis 10 Kohlenstoffatomen und 0 bis 5 Heteroatomen darstellt, was die Akkumulation von aromatischen Aldehyden in der Pflanze erhöht oder die Menge der Zimtsäure in der Pflanze erhöht, wobei die Zusammensetzung außer der aromatischen Verbindung kein zusätzliches Antioxidans enthält, wodurch wenigstens entweder das Wachstum oder die Lebensfähigkeit eines pathologischen Mikroorganismus, der die Oberfläche oder einen Teil der Pflanze besiedelt, beeinträchtigt wird, wobei der aromatische Aldehyd in einem Polymer mikroverkapselt ist.

**2.** Verfahren gemäß Anspruch 1, wobei es sich bei dem Polymer um Bienenwachs oder Carnaubawachs handelt.

**3.** Verfahren gemäß Anspruch 1, wobei das Mittel ein Balsam umfasst.

**4.** Verfahren gemäß Anspruch 3, wobei der Balsam von einem *Liquidambar*-Baum stammt.

**5.** Verfahren gemäß Anspruch 4, wobei es sich bei dem *Liquidambar*-Baum um *Liquidambar orientalis* Miller oder *Liquidambar styraciflua* handelt.

**6.** Verfahren gemäß Anspruch 3, wobei das Mittel weiterhin Zimtaldehyd oder α-Hexylzimtaldehyd oder beides umfasst.

**7.** Verfahren zur Bekämpfung des Wachstums von pathologischen Organismen auf einer Pflanze, wobei die Pflanzenoberfläche mit einer nichtphytotoxischen Zusammensetzung behandelt wird, die ein Balsam sowie eine oder mehrere aromatische Verbindungen mit der Formel

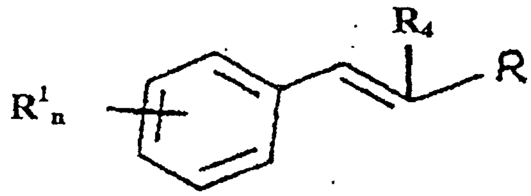

(I)

umfasst, wobei R -CHO, $CH_2OH$, -COOH oder -$COOR_5$ darstellt, n eine ganze Zahl von 0 bis 3 ist, jedes $R^1$ -OH oder einen organischen Substituenten mit 1 bis 10 Kohlenstoffatomen und 0 bis 5 Heteroatomen darstellt, wobei die Gesamtzahl an Kohlenstoff- und Heteroatomen in allen $R^1$-Substituenten der Verbindung nicht größer als 15 ist, und $R_4$ -H oder einen organischen Substituenten mit 1 bis 10 Kohlenstoffatomen darstellt und $R_5$ einen organischen Substituenten mit 1 bis 10 Kohlenstoffatomen und 0 bis 5 Heteroatomen darstellt, was die Akkumulation von aromatischen Aldehyden in der Pflanze erhöht oder die Menge der Zimtsäure in der Pflanze erhöht, wobei die Zusammensetzung außer der aromatischen Verbindung kein zusätzliches Antioxidans enthält, wodurch wenigstens entweder das Wachstum oder die Lebensfähigkeit eines pathologischen Mikroorganismus, der die Oberfläche oder einen Teil der Pflanze besiedelt, beeinträchtigt wird.

**8.** Verfahren gemäß Anspruch 7, wobei die pathologischen Organismen Blattläuse sind.

**9.** Verfahren gemäß Anspruch 7 oder 8, wobei die Zusammensetzung ein Tensid umfasst.

**10.** Verfahren gemäß einem der Ansprüche 7 bis 9, mit einem oder mehreren aromatischen Aldehyden der Formel

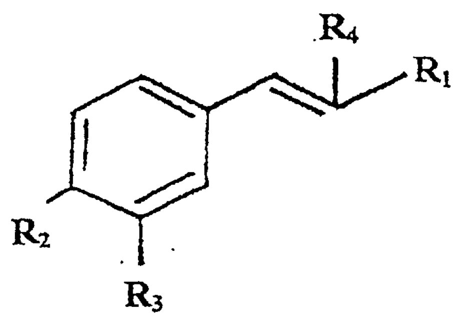

wobei $R_1$ -CHO darstellt, $R_2$ -H, -OH oder einen organischen Substituenten mit 1 bis 10 Kohlenstoffatomen darstellt und $R_3$ -H, eine Methoxygruppe oder einen organischen Substituenten mit 1 bis 10 Kohlenstoffatomen darstellt und $R_4$ -H, oder einen organischen Substituenten mit 1 bis 10 Kohlenstoffatomen darstellt.

**11.** Verfahren gemäß Anspruch 10, wobei der aromatische Aldehyd aus der Gruppe ausgewählt ist, die aus Zimtaldehyd, $\alpha$-Hexylzimtaldehyd und Coniferylaldehyd besteht.

**12.** Zusammensetzung mit einem oder mehreren aromatischen Aldehyden der Formel

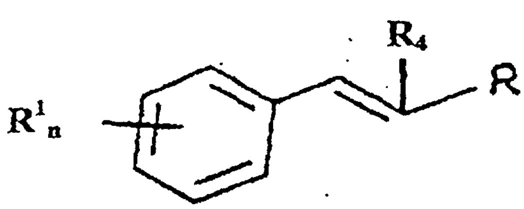

(I)

wobei R -CHO, $CH_2OH$, -COOH oder -$COOR_5$ darstellt, n eine ganze Zahl von 0 bis 3 ist, jedes $R^1$ -OH oder einen organischen Substituenten mit 1 bis 10 Kohlenstoffatomen und 0 bis 5 Heteroatomen darstellt, wobei die Gesamtzahl an Kohlenstoff- und Heteroatomen in allen $R^1$-Substituenten der Verbindung nicht größer als 15 ist, und $R_4$ -H oder einen organischen Substituenten mit 1 bis 10 Kohlenstoffatomen darstellt und $R_5$ einen organischen Substituenten mit 1 bis 10 Kohlenstoffatomen und 0 bis 5 Heteroatomen darstellt, was die Akkumulation von aromatischen Aldehyden in der Pflanze erhöht oder die Menge der Zimtsäure in der Pflanze erhöht, wobei die Zusammensetzung außer der aromatischen Verbindung kein zusätzliches Antioxidans enthält, wodurch wenigstens entweder das Wachstum oder die Lebensfähigkeit eines pathologischen Mikroorganismus, der die Oberfläche oder einen Teil der Pflanze besiedelt, beeinträchtigt wird, und die Zusammensetzung einen Balsam in einer für Pflanzen nicht phytotoxischen Zubereitung umfasst, wobei die Konzentration des Balsams ausreicht, um eine mittlere Krankheitsbekämpfung von 70% zu erhalten.

**13.** Zusammensetzung gemäß Anspruch 12, wobei die Zusammensetzung weiterhin einen oder mehrere aromatische Aldehyde der Formel

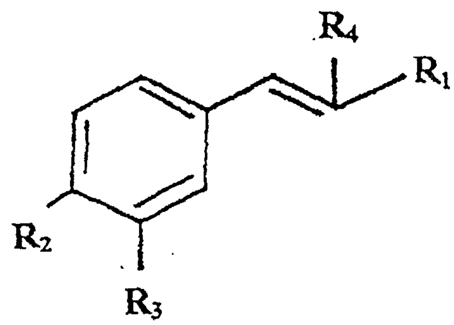

umfasst, wobei $R_1$ -CHO darstellt, $R_2$ -H, -OH oder einen organischen Substituenten mit 1 bis 10 Kohlenstoffatomen darstellt und $R_3$ -H, eine Methoxygruppe oder einen organischen Substituenten mit 1 bis 10 Kohlenstorfatomen darstellt und $R_4$ -H, oder einen organischen Substituenten mit 1 bis 10 Kohlenstoffatomen darstellt.

**14.** Zusammensetzung gemäß Anspruch 13, wobei die aromatischen Aldehyde aus der Gruppe ausgewählt sind, die aus Zimtaldehyd, α-Hexylzimtaldehyd und Coniferylaldehyd besteht.

**15.** Zusammensetzung gemäß Anspruch 12, wobei die Zubereitung eine Emulsion ist.

## Revendications

**1.** Procédé de fourniture d'une plante sensible avec une résistance accrue aux microorganismes pathologiques, ledit procédé comprenant:

la fourniture de ladite plante avec une composition non-phytotoxique comprenant au moins un composé aromatique ayant la formule

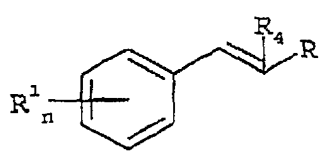

(I)

dans lequel R représente -CHO, $CH_2OH$, -COOH, ou $COOR_5$, n est un nombre entier de 0 à 3 ; chaque $R^1$ représente -OH ou un substituant organique contenant de 1 à 10 atomes de carbone et de 0 à 5 hétéroatomes, dans lequel le nombre total d'atomes de carbone et d'hétéroatomes dans tous les substituants $R^1$ dudit composé

ne dépasse pas 15 ; et $R_4$ représente -H ou un substituant organique contenant de 1 à 10 atomes de carbone, et $R_5$ représente un substituant organique contenant de 1 à 10 atomes de carbone et de 0 à 5 hétéroatomes qui augmente l'accumulation d'aldéhydes aromatiques dans ladite plante ou augmente l'acide cinnamique dans ladite plante, dans lequel ladite composition ne comprend pas un anti-oxydant supplémentaire autre que ledit composé aromatique, grâce à quoi au moins la croissance et/ou la viabilité d'un microorganisme pathologique qui colonise une surface ou une partie de ladite plante est diminuée, dans lequel ledit aldéhyde aromatique est micro-encapsulé dans un polymère.

**2.** Procédé selon la revendication 1, dans lequel ledit polymère est de la cire d'abeille ou de la cire de carnauba.

**3.** Procédé selon la revendication 1, dans lequel ledit agent comprend un balsam.

**4.** Procédé selon la revendication 3, dans lequel ledit balsam est dérivé d'un arbre Liquidambar.

**5.** Procédé selon la revendication 4, dans lequel ledit arbre Liquidambar est un Liquidambar orientalis Miller ou un Liquidambar sytraciflua.

**6.** Procédé selon la revendication 3, dans lequel ledit agent comprend de plus l'aldéhyde cinnamique et/ou de l'aldéhyde alpha-hexyl cinnamique.

**7.** Procédé de contrôle de la croissance des microorganismes pathologiques sur une plante par lequel la surface de la plante est proposée avec une composition non-phytotoxique contenant un balsam et un ou plusieurs composés aromatiques ayant la formule

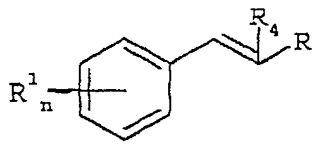

(·I)

dans lequel R représente -CHO, $CH_2OH$, -COOH, ou $COOR_5$, n est un nombre entier de 0 à 3 ; chaque $R^1$ représente -OH ou un substituant organique contenant de 1 à 10 atomes de carbone et de 0 à 5 hétéroatomes, dans lequel le nombre total d'atomes de carbone et d'hétéroatomes dans tous les substituants $R^1$ dudit composé ne dépasse pas 15 ; et $R_4$ représente -H ou un substituant organique contenant de 1 à 10 atomes de carbone, et $R_5$ représente un substituant organique contenant de 1 à 10 atomes de carbone et de 0 à 5 hétéroatomes qui augmente l'accumulation d'aldéhydes aromatiques dans ladite plante ou augmente l'acide cinnamique dans ladite plante, dans lequel ladite composition ne comprend pas un anti-oxydant supplémentaire autre que ledit composé aromatique, grâce à quoi au moins la croissance et/ou la viabilité d'un microorganisme pathologique qui colonise une surface ou une partie de ladite plante est diminuée.

**8.** Procédé selon la revendication 7, dans lequel lesdits microorganismes pathologiques sont des pucerons.

**9.** Procédé selon la revendication 7 ou 8, dans lequel ladite composition comprend un surfactant.

**10.** Procédé selon l'une quelconque des revendications 7 à 9 comprenant un ou plusieurs aldéhydes aromatiques ayant la formule

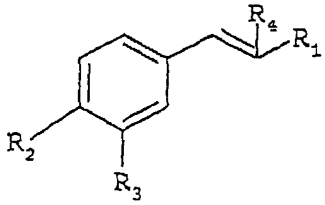

dans lequel $R_1$ représente -CHO, $R_2$ représente -H,-OH ou un substituant organique contenant de 1 à 10 atomes de carbone, et $R_3$ représente -H, un groupe méthoxy ou un substituant organique contenant de 1 à 10 atomes de carbone et $R_4$ représente -H, ou un substituant organique de 1 à 10 atomes de carbone.

**11.** Procédé selon la revendication 10, dans lequel ledit aldéhyde aromatique est choisi dans le groupe comprenant l'aldéhyde cinnamique, l'aldéhyde alpha-hexyl cinnamique et l'aldéhyde coniférylique.

**12.** Composition comprenant un ou plusieurs aldéhydes aromatiques de formule

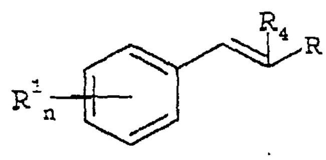

(I)

dans laquelle R représente -CHO, $CH_2OH$, -COOH, ou $COOR_5$, n est un nombre entier de 0 à 3 ; chaque $R^1$ représente -OH ou un substituant organique contenant de 1 à 10 atomes de carbone et de 0 à 5 hétéroatomes, dans lequel le nombre total d'atomes de carbone et d'hétéroatomes dans tous les substituants $R^1$ dudit composé ne dépasse pas 15 ; et $R_4$ représente -H ou un substituant organique contenant de 1 à 10 atomes de carbone, et $R_5$ représente un substituant organique contenant de 1 à 10 atomes de carbone et de 0 à 5 hétéroatomes qui augmente l'accumulation d'aldéhydes aromatiques dans ladite plante ou augmente l'acide cinnamique dans ladite plante, dans laquelle ladite composition ne comprend pas un anti-oxydant supplémentaire autre que ledit composé aromatique, grâce à quoi au moins la croissance et/ou la viabilité d'un microorganisme pathologique qui colonise une surface ou une partie de ladite plante est diminuée et ladite composition comprenant un balsam dans une formulation qui n'est pas phytotoxique pour les plantes, dans laquelle la concentration dudit balsam est suffisante pour proposer une lutte moyenne contre les parasites de 70 %.

**13.** Composition selon la revendication 12, dans laquelle ladite composition comprend de plus un ou plusieurs aldéhydes aromatiques ayant la formule:

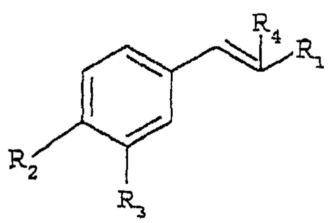

dans laquelle $R_1$ représente -CHO, $R_2$ représente -H, -OH ou un substituant organique contenant de 1 à 10 atomes de carbone, et $R_3$ représente -H, un groupe méthoxy ou un substituant organique contenant de 1 à 10 atomes de carbone, et $R_4$ représente -H, ou un substituant organique de 1 à 10 atomes de carbone.

**14.** Composition selon la revendication 13, dans laquelle lesdits aldéhydes aromatiques sont choisis dans le groupe comprenant l'aldéhyde cinnamique, l'aldéhyde alpha-hexyl cinnamique et l'aldéhyde coniférylique.

**15.** Composition selon la revendication 12, dans laquelle ladite formulation est une émulsion.